(19) Europäisches Patentamt
European Patent Office
Office européen des brevets

(11) Publication number: 0 009 227
B1

(12) # EUROPEAN PATENT SPECIFICATION

(45) Date of publication of patent specification: 01.09.82

(21) Application number: 79103475.4

(22) Date of filing: 17.09.79

(51) Int. Cl.[3]: C 07 D 489/00,
C 07 D 489/08
//C07D221/28, C07D489/02

(54) Fluorination process and fluoro analogs of hydrocodone and oxycodone.

(30) Priority: 19.09.78 US 944197
02.07.79 US 54225
19.09.78 US 944198

(43) Date of publication of application:
02.04.80 Bulletin 80/7

(45) Publication of the grant of the patent:
01.09.82 Bulletin 82/35

(84) Designated Contracting States:
BE CH DE FR GB IT LU NL

(56) References cited:
US - A - 3 137 701

CHEMICAL ABSTRACTS, vol. 87, 1977, page 599, no. 168240y
Columbus, Ohio, U.S.A.
G. SOMOGYI et al.: "Study of tosyl and mesyl series, XXI. C-6-halo derivatives of dihydrocodeine" & MAGY. KEM. FOLY. 1977, 83(7), 327—30 (Hung.)

(73) Proprietor: E.I. DU PONT DE NEMOURS AND COMPANY
Legal Department 1007 Market Street
Wilmington Delaware 19898 (US)

(72) Inventor: Boswell, George Albert
1315 Grinnel Road
Wilmington, Delaware (US)
Inventor: Henderson, Rosetta McKinley
707 Coverly Road
Wilmington, Delaware (US)

(74) Representative: von Kreisler, Alek, Dipl.-Chem. et al,
Deichmannhaus am Hauptbahnhof
D-5000 Köln 1 (DE)

(56) References cited:

CHEMICAL ABSTRACTS, vol. 77, 1972, page 529, no. 19840k
Columbus, Ohio, U.S.A.
R. BOGNAR et al.: "Reactions of tosyl and mesyl derivatives of morphines. IX. 14-Hydroxymorphine derivatives. II. Halogen compounds" & MAGY. KEM. FOLY. 1972, 78(5), 228—9 (Hung.)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 009 227 B1

(56) References cited:
CHEMICAL ABSTRACTS, vol 77, 1972, page 444, no. 152405p
Columbus, Ohio, U.S.A.
S. MAKLEIT et al.: "Conversions of tosyl and mesyl derivatives of the morphine group. XI. 14-Hydroxymorphine derivatives. II. Halogen compounds" & ACTA CHIM. (Budapest) 1972, 74(1), 111—13 (Eng.)

CHEMICAL ABSTRACTS, vol. 84, 1976, page 550, no. 90359k
Columbus, Ohio, U.S.A.
S. MAKLEIT et al.: "Conversion of tosyl and mesyl derivatives of the morphine group. XV. Synthesis of pseudocodeine tosylate and the study of its nucleophilic reaction" & MAGY. KEM. FOLY. 1975, 81(11), 517—19 (Hung.)

CHEMICAL ABSTRACTS, vol. 73, 1970, page 377, no. 131174q
Columbus, Ohio, U.S.A.
R. BOGNAR et al.: "Conversions of tosyl and mesyl derivatives of morphines. VIII. New synthesis and investigation of 6-deoxy-6-fluoroisocodeine" & MAGY. KEM. FOLY. 1970, 76(9), 461—4 (Hung.)

JOURNAL OF PHARMACEUTICAL SCIENCES vol. 65, no. 6, 1976, pages 902—904
Washington, U.S.A.
H. J. C. YEH et al.: "alpha- and beta-Halomorphides: Stereochemistry, Analgesic Potency, Toxicity, and Interaction with Narcotic Receptors in Vitro".

# Fluorination process and fluoro analogs of hydrocodone and oxycodone

*Technical Field*

This invention relates to a fluorination process for production of compounds which are useful as intermediates in syntheses of novel fluorine analogs of hydrocodone and oxycodone, the fluorine analogs exhibiting analgesic and/or narcotic antagonist properties in mammals.

*Background Art*

Morphine and codeine analgesics exhibit toxic properties or have addictive action. Considerable effort has been made to find derivatives that are free from these qualities and still have analgesic effects. Compounds which are narcotic antagonists are also useful in medicine, such as in the treatment of addicts. Fluorine derivatives of codein, i.e. 6-deoxy-6-fluorcodeines and -morphines and their dihydro derivatives have been reported by Ayer in US—A—3 137 701. They are prepared by the reaction of a 6-hydroxyalkaloid having the codeine ring structure with a fluorinating agent such as N-(2-chloro-1,1,2-trifluoroethyl)diethylamine. One such compound obtained by Ayer, and confirmed by Bognar et al., Acta Chimica Academiae Scientiarum Hungaricae *67*, 63—69 (1971), is of the formula

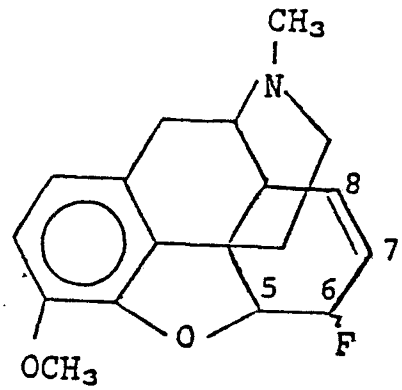

wherein the double bond is at the 7,8-position, that is, beta-gamma, relative to fluorine in the 6-position. It is well-known that fluorine substitution in organic compounds causes different biological effects and is different from chlorine, bromine or iodine substitution, as for example the 6-chloro, 7,8-double bond compound disclosed by Stork et al., J. Am. Chem. Soc. *78*, 4619 (1956).

The 6-deoxy-6-fluoromorphines and -codeines are said to be "active analgesics and central nervous system stimulants like the parent alkaloids, but in addition they possess pharmacologic advantages. Illustratively, 6-deoxy-6-fluorocodeine is a more active analgesic than codein when administered orally, and side effects are reduced. Similarly, 6-deoxy-6-fluoromorphine is an analgesic as active as morphine, but it elicits only comparatively mild side effects." No actual biological data are disclosed.

The compounds with the 7,8-double bond are allylic fluorides. Only N-methyl derivatives are contemplated in US—A—3 137 701.

Contrary to the fact that the disclosed compounds are allylic fluorides, the compounds of the instant invention are vinylic fluorides and in addition encompass variations of the nitrogen substituent, providing compounds with mixed agonist-antagonist profiles (see below, page 3, line 30—32 whereat cyclopropylmethyl and cyclobutylmethyl derivatives are disclosed). It should be noted from the last entry in the table on page 32 of the instant application that the compound of US—A 3 137 701 shown on page 1 of the instant description is relatively inactive as an analgesic (the PQW column) and has no antagonist component (the anti-straub tail column, a measure of the narcotic tendency of the compound), in marked contrast to most of the compounds of the present invention.

Diethylaminosulfur trifluoride (DAST) is a convenient reagent for replacing the carbonyl oxygen of aldehydes and ketones with two fluorine atoms. W. J. Middleton, J. Org. Chem., *40*, 574 (1975), "New Fluorinating Reagents, Dialkylaminosulfur Fluorides" and L. N. Markovskij, V. E. Pashinnik and A. V. Kirsanov, Synthesis, 787 (1973). Middleton discloses $CCl_3F$, diglyme, methylene dichloride and benzene as reaction solvents and temperatures of 25—85°C. Markovskij et al. disclose that the carbonyl compound and dialkylaminosulfur trifluoride are simply mixed in equal molar amounts and heated.

*Disclosure of the Invention*

One aspect of the invention resides in compounds of the formula

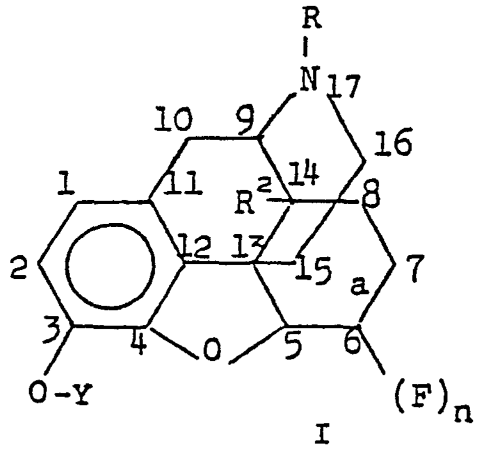

I

wherein

R is hydrogen, alkyl of 1—10 carbon atoms, allyl, methylallyl, dimethylallyl, cycloalkylmethyl of 4—7 carbon atoms, 2-furanylmethyl, 2-tetrahydrofuranylmethyl, 2-thienylmethyl, 2-thienylethyl or phenylethyl which may be ring substituted with chloro, bromo, fluoro, methoxy or 1—3 carbon atom alkyl substituents;

Y is H or $R^1$;

$R^1$ is alkyl of 1—4 carbon atoms or alkanoyl of 1—12 carbon atoms;

$R^2$ is hydrogen, hydroxy or alkanoyloxy of 1—4 carbon atoms;

n is 1 or 2; and

a is a 6,7 double bond when n is 1 and a single bond when n is 2;

and pharmaceutically acceptable salts of the compounds. Formula I and all formulas shown hereinafter are depicted by planar representations. One skilled in the art will readily understand the nonplanar aspects of these representations, for example, that the carbon-oxygen bond in the 5-position is exo or alpha. Pharmaceutically acceptable salts are those made with physiologically acceptable acids that are known in the art; such salts include the hydrochloride, sulfate, phosphate, nitrate, citrate and maleate. The invention also resides in pharmaceutically active compositions which include these compounds.

Particularly preferred are compounds of formula I wherein R is cyclopropylmethyl or cyclobutylmethyl. Those compounds wherein Y is H or acetyl are generally more active, as are those wherein $R^2$ is H. For reasons of availability and pharmacological activity, generally, n is 1 and, therefore, a is a double bond. The compounds of Examples 5—7, 11 and 12 illustrate the best mode of the invention of which Examples 5 and 11 are preferred.

Compounds of this invention can be prepared by reacting a disubstituted aminosulfur trifluoride with a mineral acid (MA), for example, HCl or $H_2SO_4$ salt of a 6-keto compound corresponding to that of formula I wherein Y is $R^1$, $R^2$ is H or alkanoyloxy of 1—4 carbon atoms, a is a single bond and $(F)_n$ is replaced by carbonyl oxygen (=O). In such process the ketone of the formula

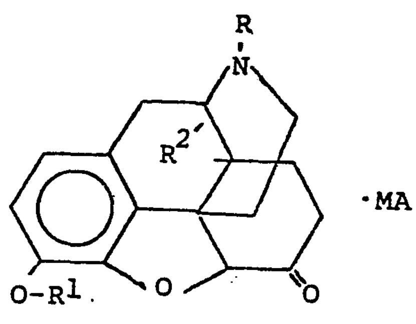

IA

wherein

R is hydrogen, alkyl of 1—10 carbon atoms, allyl, methylallyl, dimethylallyl, cycloalkylmethyl of 4—7 carbon atoms, 2-furanylmethyl, 2-tetrahydrofuranylmethyl, 2-thienylmethyl, 2-thienylethyl or phenylethyl which may be ring substituted with chloro, bromo, fluoro, methoxy or 1—3 carbon atom alkyl substituents;

$R^1$ is alkyl of 1—4 carbon atoms or alkanoyl of 1—12 carbon atoms;

$R^{2'}$ is hydrogen or alkanoyloxy of 1—4 carbon atoms; and
MA is a mineral acid.

is reacted with a disubstituted aminosulfur trifluoride fluorinating agent of the formula $R^4R^5NSF_3$ wherein each $R^4$ and $R^5$, alike or different, is a primary alkyl group of 1—4 carbon atoms; or $R^4$ and $R^5$ when taken together are $-(CH_2)_4-$, $-(CH_2)_5-$ or $-CH_2CH_2OCH_2CH_2-$; at a temperature of −40°C to +80°C; in the presence of a polar or nonpolar solvent; and recovering the fluorine-containing compound of the formula

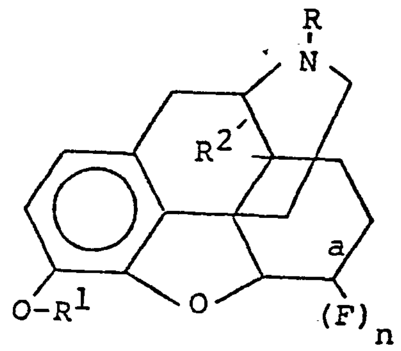
·MA

IB

wherein

R, $R^1$ and $R^{2'}$ are as defined above;
n is 1 or 2; and
a is a 6,7 double bond when n is 1 and a single bond when n is 2.

In accordance with the process of the invention, many cyclic and acylic ketones are transformed by the disubstituted aminosulfur trifluoride into vinyl fluorides as well as gem-difluorides.

When R in formula I is H (obtained as shown in Example 13), the corresponding 6-fluoride is converted to a pharmaceutically superior compound by alkylation of the HN group with RX, usually under basic conditions, wherein R is as previously defined except that it is not H and X is generally halogen (Cl, Br or I), for example, cyclopropylmethyl bromide.

In the compounds obtainable by the procedures herein, the substituent in the 3-position is generally the 3-methoxy substituent. Treatment of such compunds with a demethylating agent, such as a hydrogen halide, gives the 3-hydroxy compound from which can be prepared the corresponding alkyl ether wherein the alkyl group contains 1—4 carbon atoms, for example, the propoxy or butoxy derivative, or the corresponding ester of a $C_1$—$C_{12}$ alkanoic acid, for example, the formate, butyrate or dodecanoate, using general synthetic methods. The esters can exhibit greater potency as pharmaceuticals.

The more active compounds are preferably prepared by reacting under substantially anhydrous conditions the ketone of the formula

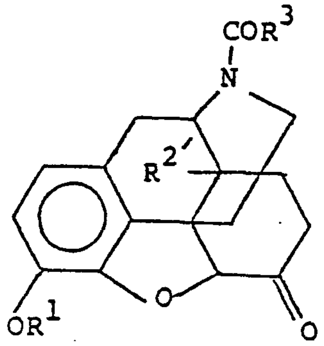

II

or

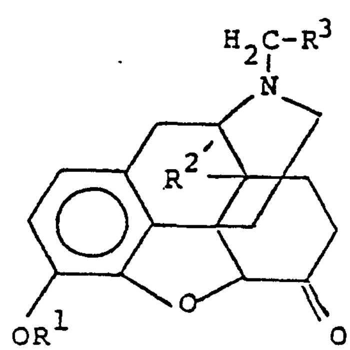
· MA

III

wherein

$R^3$ is alkyl of 1—9 carbon atoms, vinyl, 1-propenyl, isobutenyl, cycloalkyl of 3—6 carbon atoms, 2-furanyl, 2-tetrahydrofuranyl, 2-thienyl, 2-thienylmethyl or phenylmethyl which may be ring substituted with chloro, bromo, fluoro, methoxy or 1—3 carbon atom alkyl substituents;

$R^1$ is as defined above;

$R^{2'}$ is hydrogen or alkanoyloxy of 1—4 carbon atoms; and

MA represents a mineral acid,

with a disubstituted aminosulfur trifluoride of the formula $R^4R^5NSF_3$ wherein each of $R^4$ and $R^5$, alike or different, is a primary alkyl group of 1—4 carbon atoms, or $R^4$ and $R^5$ taken together are $-(CH_2)_4-$, $-(CH_2)_5-$ or $-CH_2CH_2OCH_2CH_2-$, at a temperature of −40°C to +80°C, in the presence of a polar or nonpolar solvent, and recovering the fluorine-containing compound of formula described below.

When the amide starting material of formula II is used, the aforesaid fluorination step produces an intermediate compound of the formula

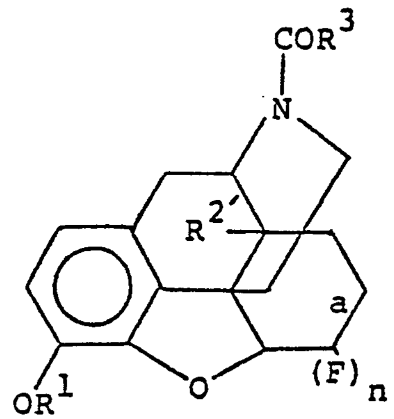

IV

wherein the symbols are as previously defined, which formula includes

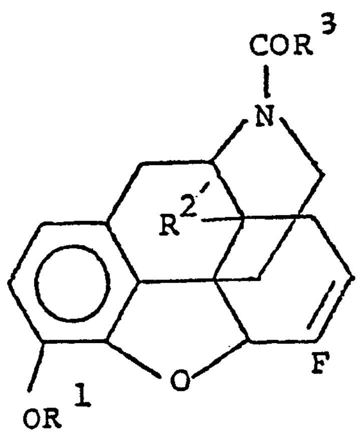

V

and

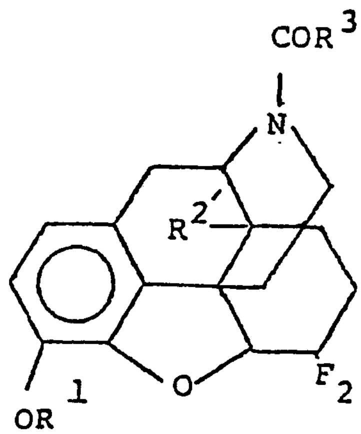

VI

This intermediate is subjected to reduction whereupon the

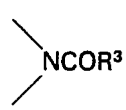

group is converted to

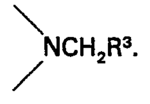

The reduction can be accomplished using conventional chemical techniques, for example, by subjecting the intermediate of formula IV to reaction with $LiAlH_4$, as exemplified in the examples below. Other reducing agents which can be used include other alkali metal hydrides, such as sodium or potassium borohydride.

When the compound of formula III is used as the starting material in the aforesaid reaction, the product will be of the formula

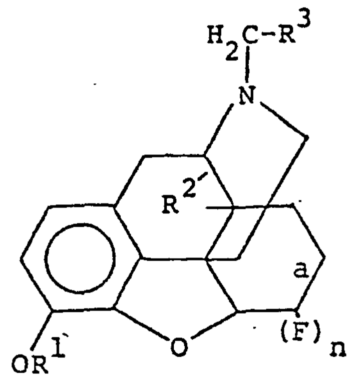

.MA

VII

wherein the symbols are as previously defined, which formula includes

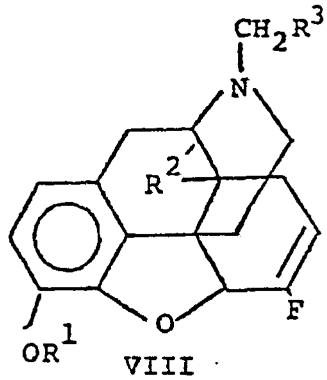

·MA

VIII

and

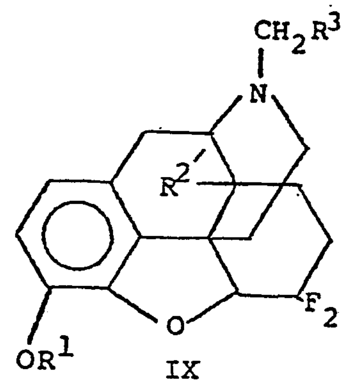

·MA

IX

Regarding formula I, when $R^2$ is to be hydroxyl in the final product, the compound subjected to fluorination should have $R^2$ as the ester (or equivalent protective group) which is subsequently converted to the hydroxyl. When $R^2$ is OH in the starting compound, the use of excess fluorinating agent, disubstituted aminosulfur trifluoride, results in the formation of a product wherein $R^2$ is F. When the starting ketone is the mineral acid salt, for example, the hydrogen sulfate of formula III, a good yield of the desired fluorinated compound is obtained. For better yields it is preferred to use the starting ketone which is the amide of formula II. It is more preferred to use such amide wherein $R^3$ has 3—8 carbon atoms.

The disubstituted aminosulfur trifluorides are known compounds. Particularly useful are diethylaminosulfur trifluoride (DAST), pyrrolidinosulfur trifluoride, morpholinosulfur trifluoride and piperidylsulfur trifluoride.

The reaction is normally carried out ina solvent medium, preferably catalytically using a highly polar catalyst. The solvent can be polar or nonpolar but it must be nonreactive with the disubstituted aminosulfur trifluoride. The use of a polar solvent tends to give more of the 6-fluoro-6,7-unsaturated codeine (n is 1) whereas the use of a nonpolar solvent generally gives more of the 6,6-difluoro compound (n is 2). By polar solvent is meant a solvent that has a high dielectric constant. Solvents which favor conversion of ketones to vinylene fluorides

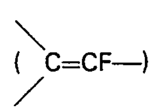

are polar and include dioxane, diethyl ether, tetrahydrofuran, ethylene glycol dimethyl ether (glyme), diethylene glycol dimethyl ether (diglyme) and triethylene glycol dimethyl ether. Nonpolar solvents, such as hydrocarbons and halogenated hydrocarbons, for example, $CCl_3F$, generally increase the amount of 6,6-difluoro (*gem-*) compound formed.

The addition of a small amount of a strong acid, such as fuming sulfuric acid, as a highly polar catalyst increases the rate of formation of the 6,7-unsaturated-6-fluoride. Other useful catalysts include strong mineral acids that, in the quantities used, do not react with the carbonyl group, with the carbon-carbon double bond or with other groups present in the codeine-type compounds, for example, perchloric, polyphosphoric and fluorosulfonic acids. The useful acids generally have a log $K_a$ of greater than −2. The amount of catalyst used is generally 0.001 to 1% by weight of the ketone starting material. The catalyst is believed to function by increasing the polarity of the reaction medium and by increasing the rate of the reaction.

The reaction is conducted, under substantially anhydrous conditions, in a vessel which is suitably glass, but a metal or ceramic vessel can be used. The reaction is conducted at −40°C to +80°C with the range 0—30°C being generally preferred. The reaction time is dependent upon the reactants and the temperature, with times of less than one hour to one week or more being useful. Pressure is not critical but ambient or autogeneous pressure is preferred.

The desired fluoro compound can be separated from the reaction mixture by conventional procedures. Preparative chromatography is a particularly useful procedure for separation and purification but other means, for example, crystallization or extraction can be used.

All temperatures reported herein are in °C.

Example 1

17-Methyl-6,7-didehydro-4,5-epoxy-6-fluoro-3-methoxymorphinan Hydrochloride (2) and 17-Methyl-6,7-dihydro-4-hydroxy-5-chloro-6-fluoro-3-methoxymorphinan Hydrochloride (3)

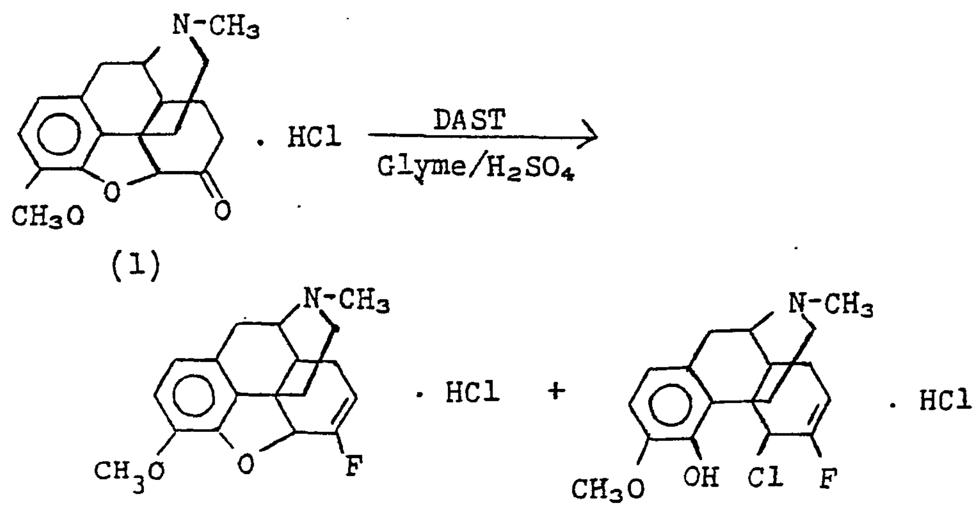


(1)

(2) (3)

A solution of 40 ml of diethylaminosulfur trifluoride (DAST) in 160 ml of glyme was added dropwise to a suspension of 8.0 g of hydrocodone hydrochloride and 1.6 ml of fuming sulfuric acid in 240 ml of glyme cooled to −78°. The reaction mixture was warmed to room temperature and stirred for 8 days. The mixture was poured over ice, made basic with sodium bicarbonate and extracted with methylene chloride. Evaporation of the methylene chloride extracts gave a viscous oil which was taken in ether and filtered. Concentration of the ether filtrate gave 4.87 g of a white solid which was chromatographed on silica. Elution with 1:1 hexane:acetone containing 1% of diethylamine gave 0.58 g of white solid 17-methyl-6,7-didehydro-4,5-epoxy-6-fluoro-3-methoxymorphinan melting at 167—169° (HCl salt 214—217°) and 2.09 g of white solid 17-methyl-6,7-dihydro-4-hydroxy-5-chloro-6-fluoro-3-methoxymorphinan melting at 169—173° (HCl salt 178—182°). The infrared spectrum showed =CF absorption at 5.91 $\mu$ with no indication of C=O. $^{19}F$ nmr ($CDCl_3$) for (3) $\delta$ −116.6 ppm; for (2) $\delta$ −116.2 ppm.

| | |
|---|---|
| HRMS (3) Calcd for $C_{18}H_{19}NO_2FCl$: | 335.1089 |
| Found: | 335.1089 |

| | |
|---|---|
| Anal. Calcd for Cl: | 19.05 |
| Found: | 19.04 |

HRMS (2) Calcd for $C_{18}H_{20}NO_2F$: 301.1497
Found: 301.1432.

Example 2

17-Methyl-4,5-epoxy-6,6-difluoro-3-methoxymorphinan Hydrochloride (4) and 17-Methyl-6,7-didehydro-4,5-epoxy-6-fluoro-3-methoxymorphinan (5)

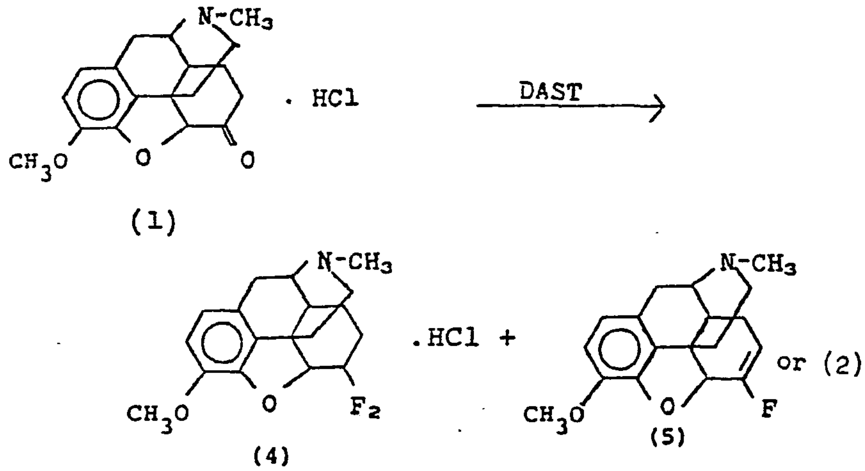


(1)

(4)

(5)

or (2)

A mixture of 10.0 g (0.029 mole) of hydrocodone hydrochloride in 200 ml of $CCl_3F$ (Freon® 11) was cooled to −78°. To this was added dropwise a solution of 50 ml of diethylaminosulfur trifluoride (DAST) in 50 ml of $CCl_3F$. The mixture was warmed to room temperature, stirred for 5 days, then poured over crushed ice. The solution was made basic with sodium bicarbonate and the layers were separated. The aqueous layer was extracted with methylene chloride and the combined organic extracts were washed with water, then brine and dried ($MgSO_4$). This gave 8.65 g of crude product as a viscous oil. A 1.6 g sample was fractionated by preparative chromatography to give 800 mg of the pure difluoro derivative 17-methyl-4,5-epoxy-6,6-difluoro-3-methoxymorphinan. A sample converted to the hydrochloride salt melted at 270—275°. $^{19}F$ nmr ($CDCl_3$) $\delta$ −91.6, −94.2, −104.1 and −106.7 ppm.

The reaction mixture also contained minor amounts of the monofluoro-$\alpha,\beta$-unsaturated compound (5) or (2).

Example 3

17-Methyl-6,7-didehydro-4,5-epoxy-6-fluoro-3-hydroxymorphinan Hydrochloride (6)

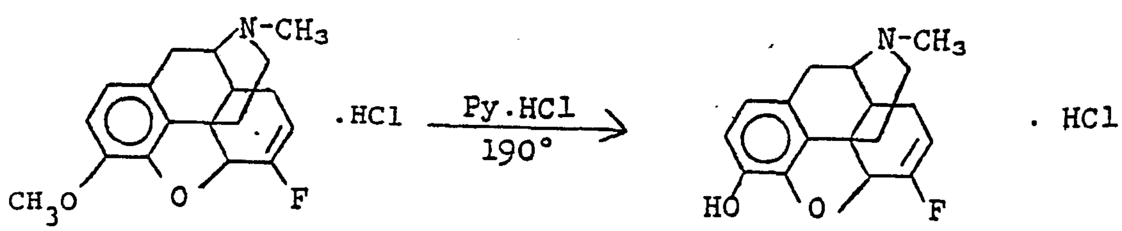


(2)

(6)

A mixture of 800 mg of the methoxy derivative 2 of Example 1 and 2.4 g of pyridine hydrochloride was heated at 190° for 3 hours, cooled and diluted with water. The aqueous solution was made basic with sodium hydroxide and extracted with methylene chloride. The methylene chloride extracts were dried ($MgSO_4$) and concentrated. The resulting oil was taken in ether and converted to the hydrochloride salt to give 200 mg of the O-demethylated derivative (6) as a white crystalline solid. High performance liquid chromatography (HPLC) showed one major component (95%).

HRMS: Calcd for $C_{17}H_{18}NO_2F$: 287.1320
Found: 287.1320.

Example 4

17-Cyclobutylcarbonyl-4,5-epoxy-6,6-difluoro-3-methoxymorphinan (8) and 17-Cyclobutylcarbonyl-6,7-didehydro-4,5-epoxy-6-fluoro-3-methoxymorphinan (9)

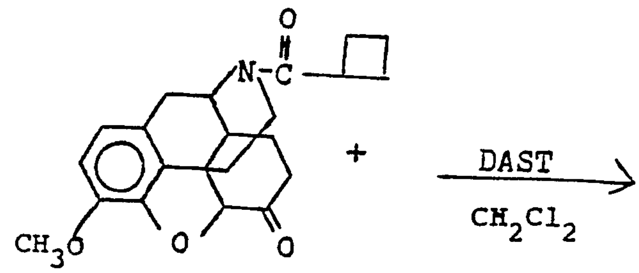


(7)

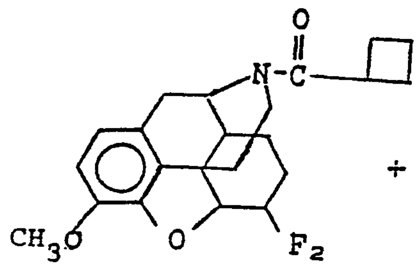

(8)

+

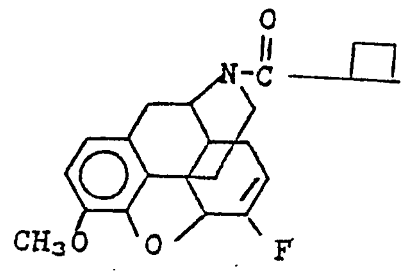

(9)

A solution of 25.0 g (68 mmoles) of the keto amide (7), prepared by reaction of cyclobutyl carbonyl chloride with the amine (11) of Example 5, in 200 ml of dry methylene chloride was cooled to −78°. To this was added dropwise a solution of 25 ml (200 mmoles) of DAST in 100 ml of dry methylene chloride. The mixture was stirred at 25° for 6 days, poured onto crushed ice and neutralized, separated, washed with water, then brine and dried over $MgSO_4$. The resulting oil was collected with ether to give 14.32 g of white solid mixture of (8) and (9), mostly (8). Concentration of the ether filtrate gave an additional 10.0 g of solid. $^{19}F$ nmr ($CDCl_3$): $\delta$ −91.6, −94.2, −104.3 and −106.9 ppm ($-CF_2$) and −116.4 ppm (=CF).

II. 17-Cyclobutylmethyl-4,5-epoxy-6,6-difluoro-3-methoxymorphinan Hydrochloride (10)

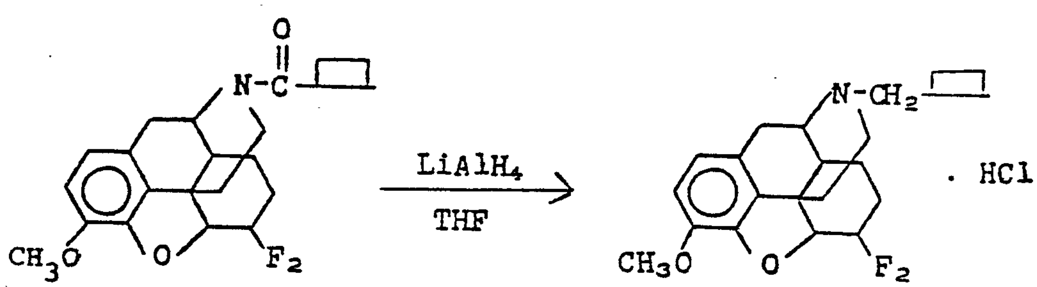


(8) (10)

To a stirred suspension of 4.5 g of $LiAlH_4$ in 200 ml of anhydrous tetrahydrofuran was added dropwise a mixture of 12.2 g of the amides (8) and (9) from part I in 100 ml of anhydrous tetrahydrofuran. The mixture was heated at reflux for 24 hours, cooled and then hydrolyzed using: 4.5 ml of water, 4.5 ml of 15% aqueous sodium hydroxide and 13.5 ml of water. This gave 6.1 g of crude amines. A 2.2 g sample was chromatographed on silica gel. Elution with methylene chloride containing 2% methanol and 1% diethylamine gave 1.5 g of 90% pure difluoride as a viscous oil. This was dissolved in ether and converted to the hydrochoride salt (10). $^{19}F$ nmr ($CDDl_3$) $\delta$ −91.7, −94.3, −104.2 and −106.8 ppm (S). Infrared spectrum showed

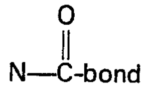

absent.

HRMS: Calcd for $C_{22}H_{27}NO_2F_2$: 375.2008
Found: 375.2003.

Example 5

17-Cyclopropylmethyl-4,5-epoxy-6,6-difluoro-3-methoxymorphinan (15) and 17-Cyclopropylmethyl-6,7-didehydro-4,5-epoxy-6-fluoro-3-methoxymorphinan (16) and Hydrochlorides

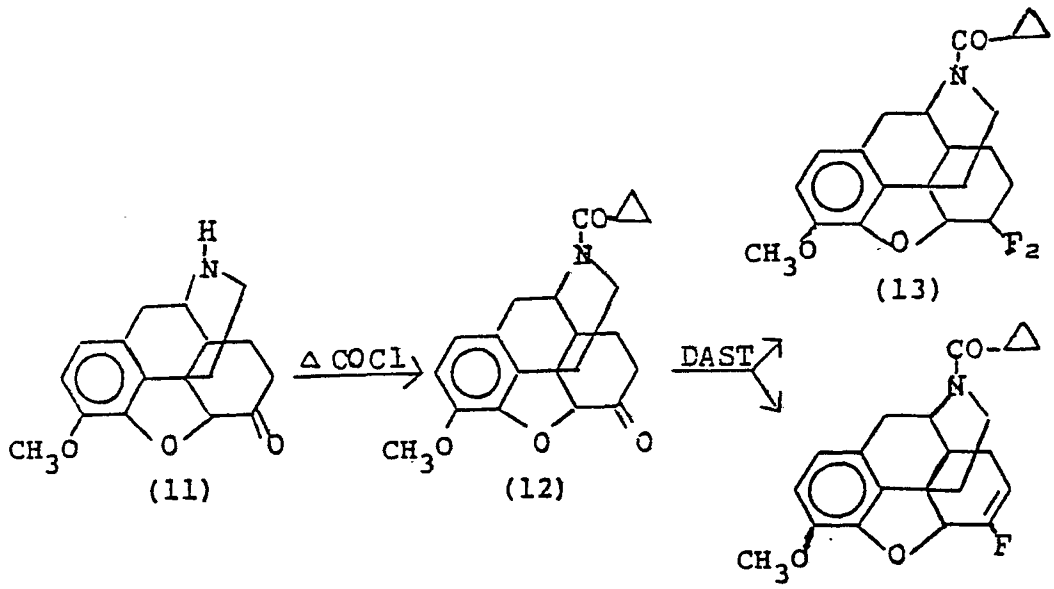


(11) (12) (13) (14)

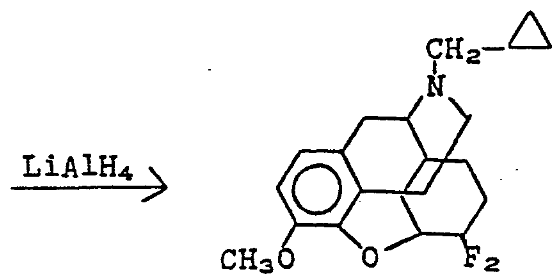


(13) (15).

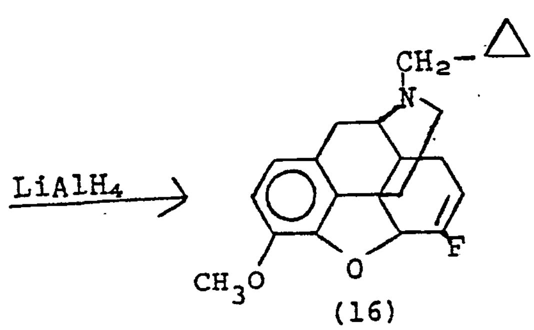


(14) (16)

The N-cyclopropylcarbonylcodone (12) was obtained by reacting 18 g of cyclopropane carbonyl chloride with 30.7 g of the amine (11) at room temperature in methylene chloride in the presence of 20 g of tetraethylamine; yield 33 g as a white solid; identical with that of Gates, J.Med.Chem. 7, 127 (1964).

When 18.5 g of the amide (12) was dissolved in 150 ml of methylene chloride and cooled and 18 ml of DAST was added, after 3 days at room temperature 19.2 g of brown oil resulted. This was dissolved in ether and filtered and the oil was chromatographed twice on silica using 90% ether-cyclohexanone with 1% methylene chloride and 0.001% water. There was obtained 4.2 g of 87% pure difluoride (13) and 6.8 g of about a 50:50 mixture of difluoride (13) and monofluoro compound (14).

The amide (13) (7 g) was dissolved in 100 ml of tetrahydrofuran and mixed with 3 g of $LiAlH_4$ in 100 ml of tetrahydrofuran. After refluxing for 16 hours, 3 ml of water was added followed by 3 ml of 15% aqueous NaOH and 9 ml of water to give 6.13 g of product which was chromatographed using 1:1 hexane:acetone containing 1% triethylamine to give 3.6 g of (15) in 96% purity by $^{19}F$ nmr or 91% by HPLC as a white solid m.p. 69—73°. The hydrochloride of the latter softened at 100° and had a mp of 123—133°. HMRS Calcd for $C_{21}H_{25}NO_2F_2$: 361.1852; Found: 361.1846.

Ten g of codone amide (12) was dissolved in 120 ml of glyme and 0.8 ml of sulfuric acid and 10 ml of DAST in 80 ml of glyme were added at −78°. After stirring at room temperature for 6 days the mixture was poured on ice, neutralized and extracted with methylene chloride. The extracts were evaporated and the residue was dissolved in ether to give 10.2 g of the 6-fluoromorphinan (14) which was chromatographed on silica using 90% ether-hexane containing 1% methylene chloride and 0.1% water. About 2.3 g of this product was dissolved in 50 ml of tetrahydrofuran and reduced with 1.1 g of $LiAlH_4$ in 50 ml of tetrahydrofuran by refluxing for 16 hours. There was obtained 1.4 g of white product (16) as the HCl salt, mp 93—97°.

Example 6

17-Cyclopropylmethyl-4,5-epoxy-6,6-difluoro-3-hydroxymorphinan (17) and Hydrochloride

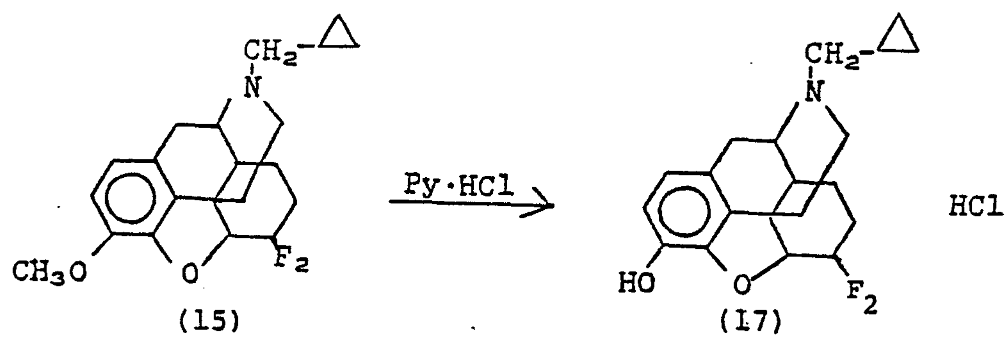


(15) (17)

The O-methyl ether (15) of Example 5 (2.8 g) was heated at 190—195° for 3 hours with 7 g of pyridine hydrochloride, diluted with water and extracted with methylene chloride; the extracted material was dissolved in toluene, filtered, concentrated, dissolved in ether and crystallized from ether-hexane by adding hexane; the resultant white powder (1 g) mp 78—84° was converted to the HCl salt (17) and purified by HPLC (96% pure), mp 312—317°. HRMS Calcd for $C_{20}H_{23}NO_2F_2$: 347.1695; Found: 347.1708.

Example 7

17-Cyclopropylmethyl-6,7-didehydro-4,5-epoxy-6-fluoro-3-hydroxymorphinan Hydrochloride (18)

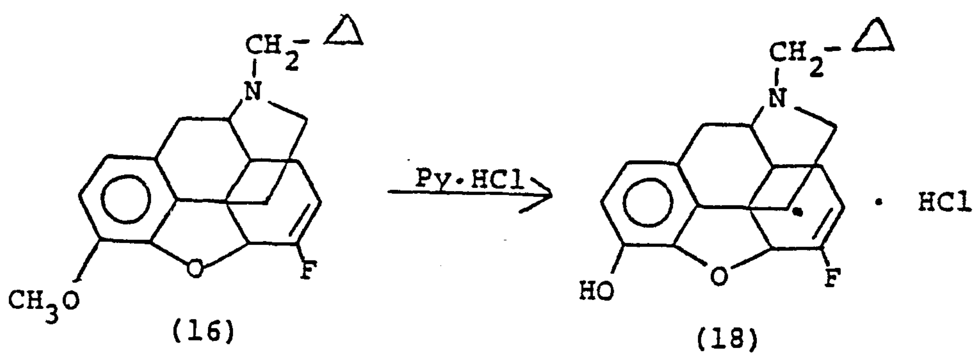


(16) (18)

The O-methyl ether (16) of Example 5 (2.9 g) was reacted with 7 g of pyridine hydrochloride as in Example 6; there was obtained a tan solid which was taken up in ether and converted to the

hydrochloride salt. The salt was recrystallized from ethanol to give 0.6 g of white solid (18) mp 285—290°. HPLC showed one major peak (94.9%).

| | |
|---|---|
| HRMS: Calcd for $C_{20}H_{22}NO_2F_2$: | 327.1627 |
| Found: | 327.1598. |

Example 8

17-Allyl-4,5-epoxy-6,6-difluoro-3-methoxymorphinan (20) and 17-Allyl-6,7-didehydro-4,5-epoxy-6-fluoro-3-methoxymorphinan (21) and Hydrochlorides

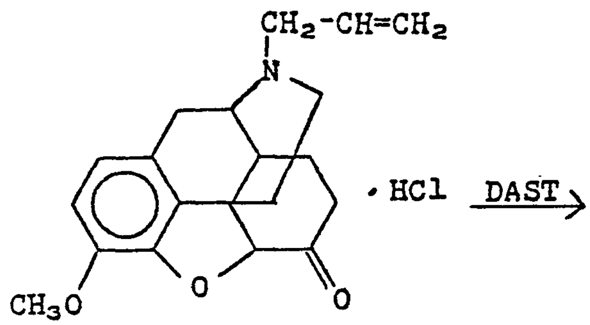


(19)

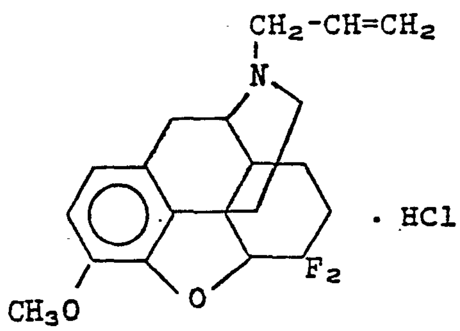


+

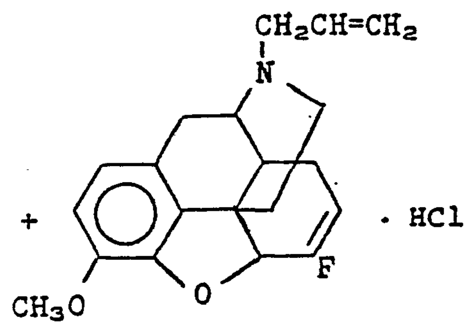


(20) (21)

To 10 g of amine (11) of Example 5 in 150 ml of dimethylformamide was added 4 g of allyl bromide and 2.8 g of $NaHCO_3$. The mixture was heated at 80—85° for 5 hours, diluted with water, extracted with ether and converted to 12.7 g of the amine salt (19). The salt was reacted with diethylaminosulfur trifluoride according to the procedure of Example 2 and the hydrochloride salts (20) and (21) thus formed were separated by chromatography as described in Example 5. $^{19}$F nmr ($CDCl_3$): $\delta$ −19.6, −94.2, −104.1 and 106.7 ppm.

Example 9

I. 17-Cyclobutylcarbonyl-4,5$\alpha$-epoxy-6-keto-14$\beta$-acetyl-3-methoxymorphinan (23)

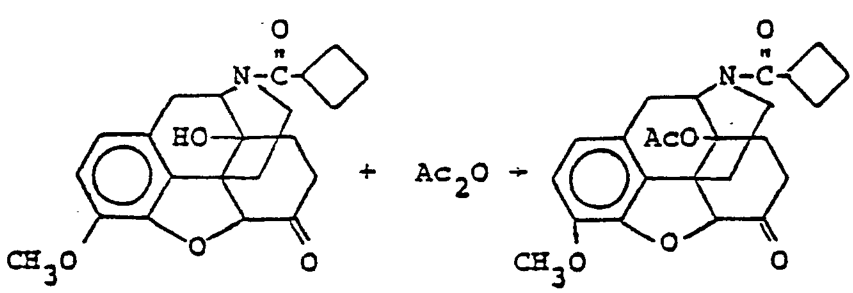


(22) (23)

A solution of 23.0 g of the morphinan (22) in 100 ml of acetic anhydride was heated at reflux for 0.5 hour cooled and then concentrated. The residue was dissolved in 200 ml of water, cooled to 0° and adjusted to pH 9 with ammonium hydroxide. A viscous oil precipitated. The water layer was decanted and the oil was dissolved in methylene chloride and dried over $Na_2SO_4$. Concentration of the methylene chloride gave 22.3 g of viscous oil. The infrared spectrum was substantially free of $OH^-$ absorption.

II. Cyclobutylcarbonyl-4,5α-epoxy-6,6-difluoro-14β-acetyl-3-methoxymorphinan (24) and 17-Cyclobutylcarbonyl-6,7-didehydro-4,5α-epoxy-6-fluoro-14-acetyl-3-methoxymorphinan (25)

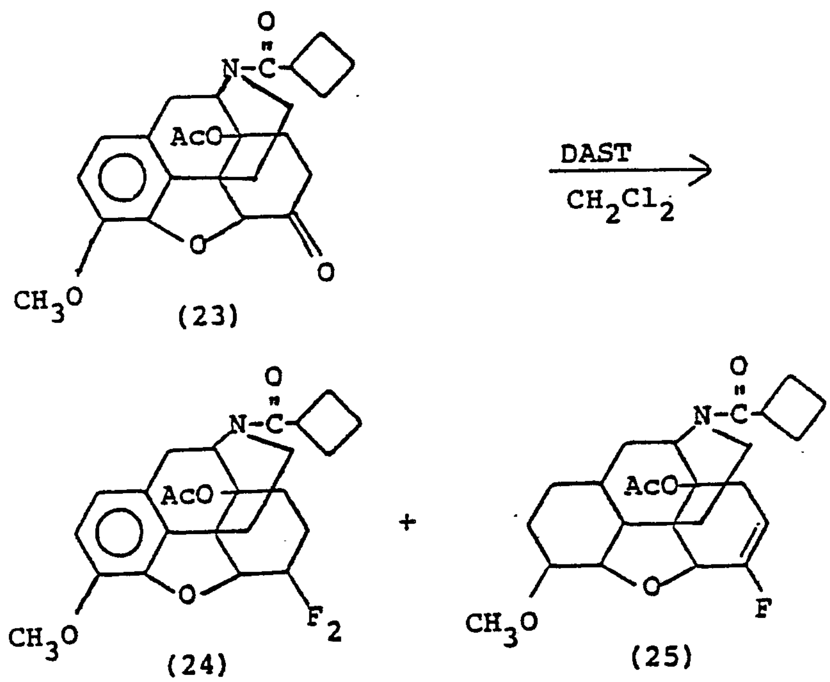

(23)

(24)

(25)

A solution of 18.0 g of the ketoamide (23) in 150 ml of dry methylene chloride was cooled to −78°. A solution of 18 ml of DAST in 150 ml of methylene chloride was added dropwise. The reaction mixture was stirred at room temperature for three days, poured over ice and neutralized with sodium bicarbonate. The organic layer was separated, washed with brine and dried ($K_2CO_3$). This gave 16.6 g of the crude fluorides as a viscous oil.

| | |
|---|---|
| HRMS: Calcd for $C_{24}H_{27}NO_5F_2$: | 447.1856 |
| Found: | 447.1846 |
| Calcd for $C_{24}H_{26}NO_5F$: | 427.1793 |
| Found: | 427.1752. |

The above fluoro derivatives (24) and (25) were separated using HPLC. Elution with hexane-acetone-triethylamine gave 8 g of the difluoride (24) as a colorless viscous oil. $^{19}F$ nmr ($CDCl_3$) δ −91.3, −93.9 and 102.3, 104.4 ppm (—$CF_2$).

III. 17-Cyclobutylmethyl-4,5α-epoxy-6,6-difluoro-14β-hydroxy-3-methoxymorphinan Hydrochloride (26)

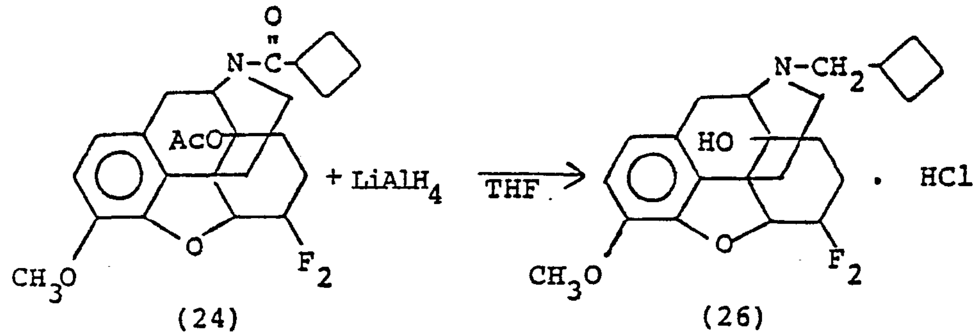


(24) (26)

An 8 g sample of the difluoro amide (24) was reduced as in Example 4, II. This gave 4.5 g of tan solid which was dissolved in ether; some insoluble solid was filtered off. Concentration of the ether-soluble material gave 2.6 g of pale yellow solid which was taken in ether and decolorized by use of charcoal to give a white solid melting at 162—164°. When converted to the hydrochloride salt (in ether) the difluoromorphinan (26) melted at 266—269°. Its infrared spectrum showed hydroxyl absorption at 3400 $cm^{-1}$ but no amide or carbonyl absorption.

Example 10

17-Cyclopropylmethyl-4,5α-epoxy-6,6-difluoro-14β-hydroxy-3-methoxymorphinan Hydrochloride (28)

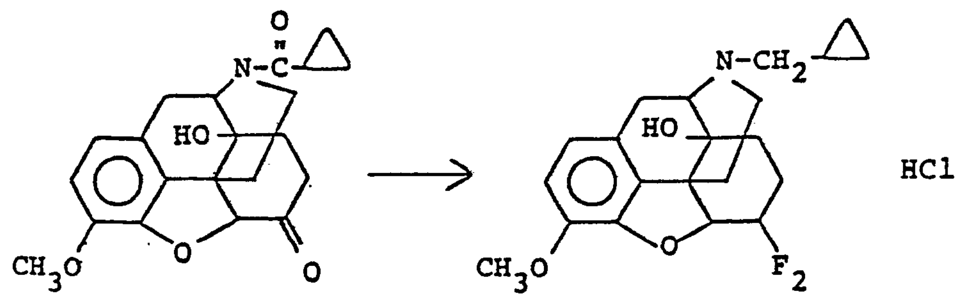


(27) (28)

Using the procedures described hereinabove, starting with (27) and acetylating, fluorinating with DAST, recovering the difluoride and hydrolyzing and reducing, as in Example 9, gave the hydrochloride salt (28) which melted at 248—252°.

| | |
|---|---|
| Anal. Calcd for $C_{24}H_{25}NO_3F_2$: | C, 66.83; H, 6.68; N, 3.71 |
| Found: | C, 66.47; H, 6.64; N, 3.97. |

Example 11

17-Cyclopropylmethyl-6,7-didehydro-4,5α-epoxy-6-fluoro-3-acetoxymorphinan (29)

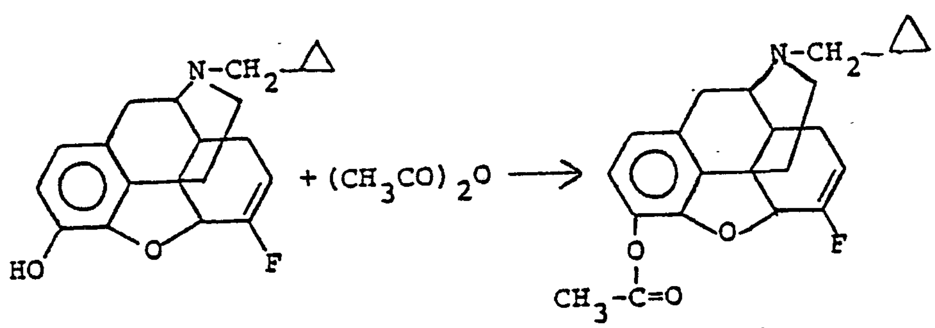


(18) (29)

A solution of 3.0 g of the crude phenol (18) of Example 7 in 25 ml of acetic anhydride was heated at reflux for 0.5 hour and then concentrated. The residue was treated with ice water and made basic

with ammonia. The aqueous base was extracted with methylene chloride, treated with decolorizing charcoal and dried ($K_2CO_3$). Removal of the solvent gave 3.6 g of tan oil which was recrystallized from hexane-ether to give 0.84 g of the acetoxy derivative (29) as a white solid melting at 86—88°. The infrared spectrum showed strong carbonyl absorption at 1730 $cm^{-1}$.

| | |
|---|---|
| Anal. Calcd for $C_{22}H_{24}NO_3F$: | C, 71.53; H, 6.45; N, 3.79 |
| Found: | C, 71.58; H, 6.64; N, 3.75. |

A sample of the base was dissolved in ether and converted to the hydrochloride salt which melted at 139° (dec.).

Example 12

17-Cyclopropylmethyl-4,5$\alpha$-epoxy-6,6-difluoro-3-acetoxymorphinan (30)

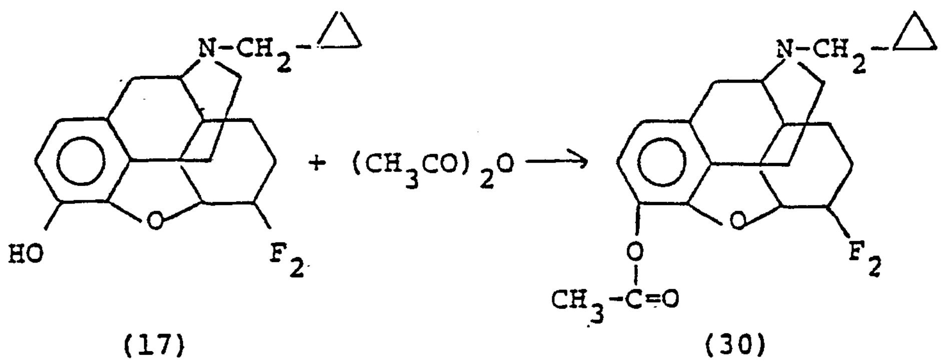


(17) (30)

The procedure of Example 11 was used to prepare the acetoxy derivative of the corresponding difluoride. The difluoride (30) was obtained as a white solid melting at 128—130°.

| | |
|---|---|
| Anal. Calcd for $C_{22}H_{25}NO_3F_2$: | C, 67.85; H, 6.47; N, 3.60 |
| Found: | C, 67.23; H, 6.42; N, 3.52, |
| HRMS: Calcd for $C_{22}H_{25}NO_3F_2$: | 389.1801 |
| Found: | 389.1842. |

Example 13

6-Fluoro- (32) and 6,6-difluorodihydronorcodeinone (33)

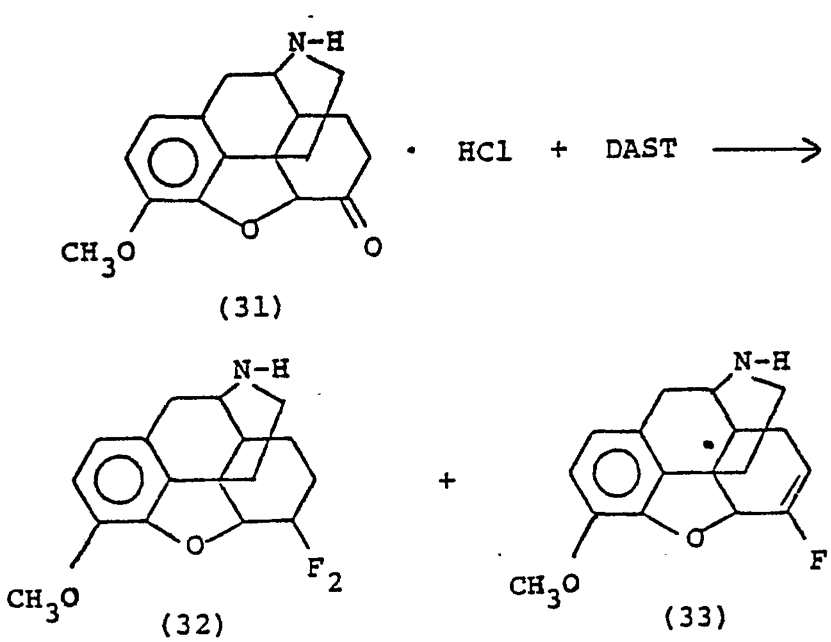


(31)

(32) + (33)

A mixture of 13.5 g of the nordihydrocodeinone hydrochloride (31) in 250 ml of methylene chloride was treated with 13 ml of DAST using the procedures described hereinabove. After work-up the residue was taken in ether and filtered. This gave 6.7 g of a mixture of the fluoro derivatives (32) and (33) as a tan solid. These compounds were separated by chromatography as described in Example 1. $^{19}F$ nmr −93.13, −105.57 ppm (difluoride) and −116.12 ppm (vinyl).

| | |
|---|---|
| HRMS: Calcd for $C_{17}H_{19}NO_2F_2$: | 307.1383 |
| Found: | 307.1353 |

| | |
|---|---|
| HRMS: Calcd for $C_{17}H_{18}NO_2F$: | 287.1320 |
| Found: | 287.1292. |

The fluoronorcodeinones (32) and (33) of this example are valuable intermediates in synthesis of N-substituted compounds of formula I. For example, they can be alkylated with RX, wherein X is Cl or Br, in a solvent in the presence of a base. Suitable alkylating agents include cyclohexylmethyl bromide, dimethylallyl, chloride, 2-thienylmethyl chloride, p-methoxyphenylethyl bromide and cyclopropylmethyl chloride; these give, respectively, the corresponding N-cyclohexylmethyl, dimethylallylic, thienylmethyl and phenylethyl compounds.

When the general procedures of Examples 4 and 5 are carried out using compounds corresponding to (7) and (12) but where $R^3$ is $CH_2C_6H_5$, $C_6H_{11}$ or $(CH_2)_9H$, there are obtained the corresponding compounds N-phenylethyl-6,7-didehydro-4,5$\alpha$-epoxy-6-fluoro-3-methoxymorphinan, N-phenylethyl-4,5$\alpha$-epoxy-6,6-difluoro-3-methoxymorphinan and the N-cyclohexylmethyl- or N-*n*-decylmorphinan.

Starting with N-butyl- or N-dimethylallylcodeine and using the procedures of Examples 1 and 2, the corresponding N-butyl and N-dimethylallyl substituted compounds are obtained. Similarly, N-(2-furanylmethyl), N-(2-thienylmethyl) and N-(2-thienylethyl) substituted compounds corresponding to those of Example 5 can be prepared and used as described above.

When the N-cyclobutanoylnoroxycodone-14-acetate is used in the general procedures of Example 9, the 6,6-difluoro- and 6-fluoro-Δ6,7-cyclobutylmethylnoroxycodone-14-acetate are obtained. Hydrolysis of the 14-acetate compounds gives the 14-hydroxy compounds. The corresponding 14-butanoyl, 14-propionyl and 14-formyl compounds can be prepared by suitable acylation of the 14-hydroxy compounds with appropriate alkanoyl halides.

*Pharmaceutical Salts*

Pharmaceutically suitable acid addition salts of the compounds of this invention include those made with physiologically acceptable acids that are known in the art; such salts include hydrochloride, sulfate, phosphate, nitrate, citrate, and maleate.

*Utility*

The process of this invention provides compounds which are useful intermediates in the synthesis of the N-substituted compounds, of this invention, which are active pharmaceutically as analgesics and/or narcotic antagonists.

*Dosage Forms and Use*

The compounds of this invention can be administered as analgesic agents to alleviate pain or as narcotic antagonists by any means that produces contact of the active agent with the agent's site of action in the body of a mammal. They can be administered either as individual therapeutic agents or in a combination of therapeutic agents, generally with a pharmaceutical carrier. The dosage depends upon: the pharmacodynamic characteristics of the particular agent, and its mode and route of administration; the age, health, and weight of the recipient; the nature and extent of the symptoms; the kind of concurrent treatment; the frequency of treatment; and the effect desired. Usually a daily dosage of active ingredient can be about 0.05 to 100 milligrams per kilogram of body weight in divided doses 2 to 4 times a day or in sustained release form.

Dosage forms (compositions) suitable for internal administration can contain about 25 milligrams to about 75 milligrams of active ingredient per dosage unit. In the pharmaceutical compositions herein the active ingredient will ordinarily be present in an amount of about 0.5—95% by weight based on the total weight of the composition.

The active ingredient can be administered orally in solid dosage forms such as capsules, tablets, and powders, or in liquid dosage forms such as elixirs, syrups and suspensions. It can also be administered parenterally in sterile liquid dosage forms or rectally in the form of suppositories.

Capsules or tablets contain the active ingredient and powdered carriers, such as lactose, sucrose, mannitol, starch, cellulose derivatives, magnesium stearate and stearic acid; they can be manufactured as sustained release products.

In general, suitable carriers for parenteral solutions include water, a suitable oil, saline, aqueous dextrose (glucose), and related sugar solutions, and glycols such as propylene glycol or polyethylene glycols. Solutions for parenteral administration contain, preferably: a water-soluble salt of the active

ingredient, for example, the hydrochloride; suitable stabilizing agents; buffer substances; anti-oxidizing agents such as ascorbic acid; and preservatives such as benzalkonium chloride, methyl- or propylparaben and chlorobutanol.

Suppositories contain the active ingredient in a suitable oleaginous or water-soluble base. The oleaginous class includes cocoa butter and fats having similar properties; the water-soluble class includes polyethylene glycols.

Suitable pharmaceutical carriers are described in *Remington's Pharmaceutical Sciences*, E. W. Martin, a standard reference text in this field.

Useful pharmaceutical dosage-forms for administration of the compounds of this invention can be illustrated by the following:

*Capsules*

A large number of unit capsules are prepared by filling standard two-piece, hard gelatin capsules, each with 50 milligrams of powdered active ingredients, 110 milligrams of lactose, 32 milligrams of talc and 8 milligrams of magnesium stearate.

A mixture of active ingredient in soybean oil is prepared and injected by means of a positive displacement pump into gelatin to form soft gelatin capsules containing 50 milligrams of the active ingredient. The capsules are washed in petroleum ether and dried.

*Tablets*

A large number of tablets are prepared by conventional procedures so that the dosage unit is 50 milligrams of active ingredient, 7 milligrams of ethyl cellulose, 0.2 milligram of colloidal silicon dioxide, 7 milligrams of magnesium stearate, 11 milligrams of microcrystalline cellulose, 11 milligrams of cornstarch and 98.8 milligrams of lactose. Appropriate coatings may be applied to increase palatability or delay absorption.

*Injectable*

A parenteral composition suitable for administration by injection is prepared by stirring 1.5% by weight of active ingredient in 10% by volume of propylene glycol and water. The solution is sterilized by filtration.

Another parenteral composition suitable for administration by injection is prepared by dissolving 1% by weight of active ingredient in sodium chloride injection U.S.P. XV and adjusting the pH of the solution to between 6 and 7. The solution is sterilized by filtration.

*Suspension*

An aqueous suspension is prepared for oral administration so that each 5 milliliters contain 10 milligrams of finely divided active ingredient, 500 milligrams of acacia, 5 milligrams of sodium benzoate, 1.0 gram of sorbitol solution, U.S.P., 5 milligrams of sodium saccharin and 0.025 millilitre of vanilla tincture.

*Test Results*

A standard procedure for detecting and comparing the analgesic activity of compounds in this series for which there is good correlation with human efficacy is the standard phenylquinone writhing test modified from Siegmund, et. al., *Proc. Soc. Exp. Biol. Med 95,* 729 (1957). A test compound suspended in 1% methylcellulose was given orally to fasted (17—21 hours) female white mice, 5—20 animals per double blind test. Aqueous (0.01% phenyl-*p*-benzoquinone) phenylquinone was injected intraperitoneally at 24 minutes later using 0.20 ml per mouse. Commencing at 30 minutes after the oral administration of the test compound, the mice were observed for 10 minutes for a characteristic stretching or writhing syndrome which is indicative of pain induced by phenylquinone. The effective analgesic dose for 50% of the mice (ED 50) was calculated by the moving average method of Thompson, U.R., *Bact. Rev. 11* 115—145 (1947). This is reported as PQW $ED_{50}$ in the table below.

Some of the compounds of this invention are particularly useful as analgesics by subcutaneous injection; for example, the compound (29) of Example 11 had an $ED_{50}$ of 0.0016 fifteen minutes after injection.

Narcotic analgesics produce in mice an erection and arching of the tail (90° or more) which is referable to spinal cord stimulation. This Straub tail reaction is not produced by other analgesics, including the narcotic antagonists.

The method used was modified from Shemano (Shemano, I., and Wendel, H., *Tox. Appl. Pharm. 6,* 334—9 (1964)). $CF_1S$ female mice (18—21 g), 10—20 mice per dose were injected subcutaneously with log scaled doses of analgesic in 0.9% saline or 0.45% saline containing 0.5% methylcellulose. A positive Straub tail response was recorded if a tail was erected for 3 seconds at any time within 25 minutes after dosing. A quantal Straub tail $ED_{50}$ was calculated by the moving average method and is an indication of physical dependence.

The narcotic antagonist (anti-Straub tail) property of the compounds is estimated by their ability to prevent morphine-induced Straub tail in mice. In this test the compound is injected intraperitoneally

into mice and 10 minutes later 53 mg/kg of morphine sulfate is given subcutaneously. Prevention of the induction of a 90° Straub tail for minutes after the morphine sulfate injection is considered to indicate narcotic antagonism in the compound tested.

The following table shows the activity in mg/kg exhibited by various compounds including those of this invention (in the hydrochloride salt form to provide solubility), when tested by the procedures given above. A value of 135 mg/kg or higher is considered to indicate an inactive compound.

| | | MOUSE $ED_{50}$ VALUES | | |
|---|---|---|---|---|
| EXAMPLE | COMPOUND NUMBER | PQW | STRAUB TAIL | ANTISTRAUB TAIL |
| 1 | 2 | 8.5 | 26.0 | 94.0 |
| 2 | 4 | 2.7 | 10.0 | > 81.0 |
| 3 | 6 | 8.0 | 17.0 | N.T. |
| 4 | 10 | 2.9* | > 81.0 | 4.1 |
| 5 | 15 | 2.2* | > 54.0 | 0.15 |
| 5 | 16 | 18.0 | >135.0 | 0.21 |
| 6 | 17 | 2.6* | > 54.0 | 0.003 |
| 7 | 18 | 7.2 | >135.0 | 0.18 |
| 9 | 26 | 26.0 | >135.0 | 10.8 |
| 11 | 29 | 1.3 | > 18 | 0.0022 |
| 12 | 30 | 2.2 | > 18 | 0.0031 |
| Codeine $PO_4$ | | 4.7* | 202.0 | >140.0 |
| Morphine $SO_4$ | | 3.0* | 48.0 | N.T. |
| US—A—3,137,701 (6-fluoro-7,8 double bond) | | 32.0 | 63.0 | >162.0 |

> = greater than
N.T.= Not Tested
*Peak time value (i.e., maximum activity within 30 min of administration).

The Straub Tail Antagonism test was modified by giving test drugs (in 1% methyl cellulose with 1.25% "Tween 80") subcutaneously, followed by intraperitoneal injection of morpholine sulfate (at 35 mg/kg) 5 minutes after the test drug. After 15 minutes, the animals were observed for 5 minutes for evidence of Straub tail. ED 50's were calculated as above with a 95% confidence limit as described by Litchfield, J. Pharmacol. Exp. Ther. *96*, 99 (1949). When compound (17) of Example 6 was tested, the $ED_{50}$ was 0.0031 mg/kg; the value for Pentazocine was 1.9 mg/kg.

**Claims**

1. Compound of the formula

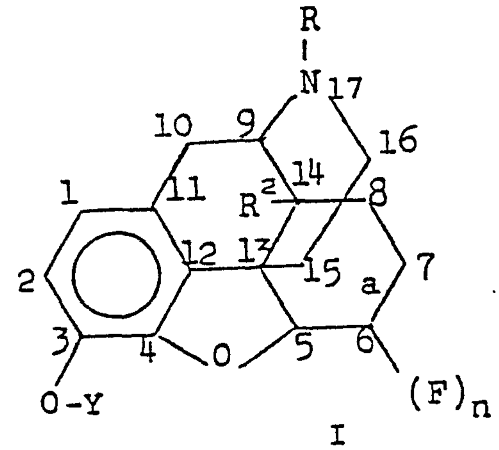

I

wherein

R is selected from the group consisting of hydrogen, alkyl of 1—10 carbon atoms, allyl, methylallyl, dimethylallyl, cycloalkylmethyl of 4—7 carbon atoms, 2-furanylmethyl, 2-tetrahydrofuranylmethyl, 2-thienylmethyl, 2-thienylethyl and phenylethyl which may be ring substituted with chloro, bromo, fluoro, methoxy or 1—3 carbon alkyl substituents;

Y is H or R';

R' is selected from the group consisting of alkyl of 1—4 carbons and alkanoyl of 1—12 carbons;

$R^2$ is selected from the group consisting of hydrogen, hydroxy and alkanoyloxy of 1—4 carbons;

n is 1 or 2; and

a is a 6,7 double bond when n=1, and a single bond when n=2;

and pharmaceutically acceptable salts of the compound.

2. The compound of Claim 1 in a pharmaceutical carrier in an amount of 0.5 to 95% by weight based on the total weight of the composition.

3. The compound of Claim 1 wherein R is cyclopropylmethyl.

4. The compound of Claim 1 wherein R is cyclobutylmethyl.

5. 17-Methyl-6,7-didehydro-4,5-epoxy-6-fluoro-3-methoxymorphinan.

6. 17-Methyl-4,5-epoxy-6,6-difluoro-3-methoxymorphinan.

7. 17-Methyl-6,7-didehydro-4,5-epoxy-6-fluoro-3-methoxymorphinan.

8. 17-Methyl-6,7-didehydro-4,5-epoxy-6-fluoro-3-hydroxymorphinan.

9. 17-Cyclobutylmethyl-4,5-epoxy-6,6-difluoro-3-methoxymorphinan.

10. 17-Cyclopropylmethyl-6,7-didehydro-4,5-epoxy-6-fluoro-3-acetoxymorphinan.

11. 17-Cyclobutylmethyl-4,5$\alpha$-epoxy-6,6-difluoro-3-acetoxymorphinan.

12. 17-Cyclopropylmethyl-4,5-epoxy-6,6-difluoro-3-methoxymorphinan.

13. 17-Cyclopropylmethyl-6,7-didehydro-4,5-epoxy-6-fluoro-3-methoxymorphinan.

14. 17-Cyclopropylmethyl-4,5-epoxy-6,6-difluoro-3-hydroxymorphinan.

15. 17-Cyclopropylmethyl-6,7-didehydro-4,5-epoxy-6-fluoro-3-hydroxymorphinan.

16. 17-Allyl-4,5-epoxy-6,6-difluoro-3-methoxymorphinan.

17. 17-Allyl-6,7-didehydro-4,5-epoxy-6-fluoro-3-methoxymorphinan.

18. 17 - Cyclobutylmethyl - 4,5$\alpha$ - epoxy - 6,6 - difluoro - 14$\beta$ - hydroxy - 3 - methoxymorphinan hydrochloride.

19. 17 - Cyclopropylmethyl - 4,5$\alpha$ - epoxy - 6,6 - difluoro - 14$\beta$ - hydroxy - 3 - methoxymorphinan hydrochloride.

20. Process of reacting under substantially anhydrous conditions the ketone of the formula

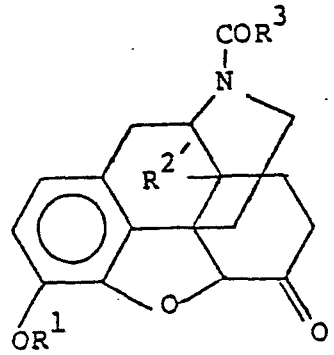

II

or

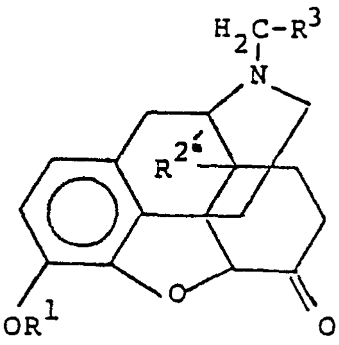

III

· MA

wherein

R[1] is an alkyl of 1—4 carbon atoms or alkanoyl of 1 to 12 carbon atoms;

$R^{2'}$ is hydrogen, or alkanoyloxy of 1—4 carbon atoms;

$R^3$ is alkyl of 1—9 carbon atoms, vinyl, 1-propenyl, isobutenyl, cycloalkyl of 3—6 carbon atoms, 2-furanyl, 2-tetrahydrofuranyl, 2-thienyl, 2-thienylmethyl or phenylmethyl which may be ring substituted with chloro, bromo, fluoro, methoxy or 1—3 carbon atom alkyl substituents; and

MA is a mineral acid,

with a disubstituted aminosulfur trifluoride fluorinating agent of the formula $R^4R^5NSF_3$ wherein each $R^4$ and $R^5$, alike or different, is a primary alkyl group of 1—4 carbon atoms; or $R^4$ and $R^5$ when taken together are $—(CH_2)_4—$, $—(CH_2)_5—$ or $—CH_2CH_2OCH_2CH_2—$; at a temperature of −40°C to +80°C; in the presence of a polar or nonpolar solvent; and recovering the fluorine-containing compound of the formula

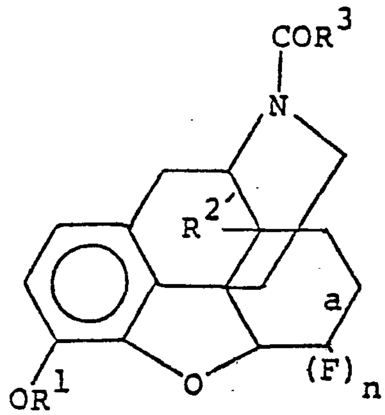

IV

when the ketone is of formula II, and of the formula

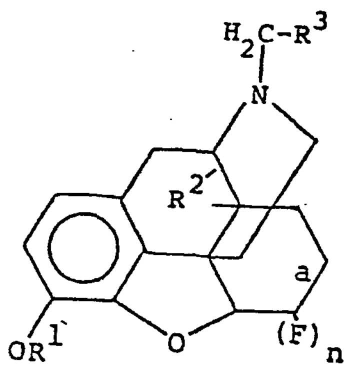

VII

when the ketone is of formula III,

wherein said formulas IV and VII

$R^1$, $R^{2'}$, $R^3$ and MA are as defined above;

n is 1 or 2; and

a is a 6,7 double bond when n is 1 and a single bond when n is 2.

21. The process of Claim 20 wherein the ketone of formula III is reacted.

22. The process of Claim 20 wherein the ketone of formula II is reacted and the fluorine-containing compound thus produced is reduced to convert the

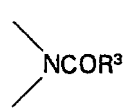

group to

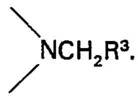

23. The process of Claim 22 wherein the reduction is effected with $LiAlH_4$.

24. The process of Claim 20 wherein the starting ketone is hydrocodone hydrochloride, the fluorinating agent is diethylaminosulfur trifluoride, and the solvent is the polar solvent ethylene glycol methyl ether.

25. The process of Claim 20 wherein the flourinating agent is diethylaminosulfur trifluoride and the solvent is fluorotrichloromethane.

26. The process of Claim 20 wherein the fluorinating agent is diethylaminosulfur trifluoride and the solvent is the polar solvent methylene chloride.

27. Process of reacting under substantially anhydrous conditions the ketone of the formula

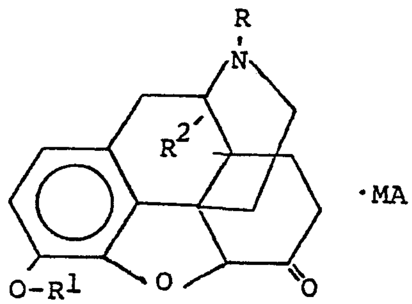

IA

wherein

R is hydrogen, alkyl of 1—10 carbon atoms, allyl, methylallyl, dimethylallyl, cycloalkylmethyl of 4—7 carbon atoms, 2-furanylmethyl, 2-tetrahydrofuranylmethyl, 2-thienylmethyl, 2-thienylethyl or phenylethyl which may be ring substituted with chloro, bromo, fluoro, methoxy or 1—3 carbon atom alkyl substituents;

$R^1$ is alkyl of 1—4 carbon atoms or alkanoyl of 1—12 carbon atoms;

$R^{2'}$ is hydrogen or alkanoyloxy of 1—4 carbon atoms; and

MA is a mineral acid,

with a disubstituted aminosulfur trifluoride fluorinating agent of the formula $R^4R^5NSF_3$ wherein each $R^4$ and $R^5$, alike or different, is a primary alkyl group of 1—4 carbon atoms; or $R^4$ and $R^5$ when taken together are $-(CH_2)_4-$, $-(CH_2)_5-$ or $-CH_2CH_2OCH_2CH_2-$; at a temperature of −40°C to +80°C; in the presence of a polar or nonpolar solvent; and recovering the fluorine-containing compound of the formula

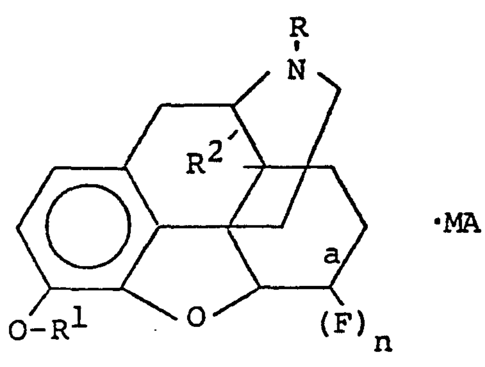

IB

wherein

R, $R^1$ and $R^{2'}$ are as defined above;

n is 1 or 2; and

a is a 6,7 double bond when n is 1 and a single bond when n is 2.

28. Compound of the formula

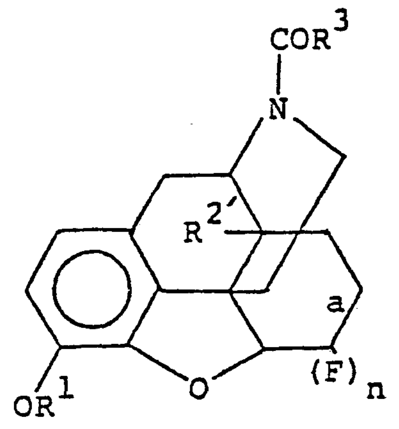

IV

wherein

$R^1$ is alkyl of 1—4 carbon atoms or alkanoyl of 1—12 carbon atoms;

$R^{2'}$ is hydrogen or alkanoyloxy of 1—4 carbon atoms;

$R^3$ is alkyl of 1—9 carbon atoms, vinyl, 1-propenyl, isobutenyl, cycloalkyl of 3—6 carbon atoms, 2-furanyl, 2-tetrahydrofuranyl, 2-thienyl, 2-thienylmethyl or phenylmethyl which may be ring-substituted with chloro, bromo, fluoro, methoxy, or 1—3 carbon atoms alkyl substituents;

n is 1 or 2; and

a is a 6,7 double bond when n is 1 and a single bond when n is 2.

**Revendications**

1. Composé de la formule:

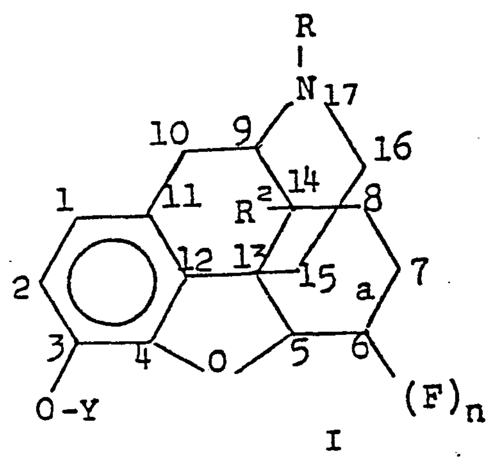

I

dans laquelle

R est choisi parmi l'hydrogène, un groupe alcoyle de 1 à 10 atomes de carbone, allyle, méthylallyle, diméthylallyle, cycloalcoylméthyle de 4 à 7 atomes de carbone, 2-furanylméthyle, 2-tétrahydrofuranylméthyle, 2-thiénylméthyle, 2-thiényléthyle et phényléthyle qui peut être substitué au noyau par des substituants chloro, bromo, fluoro, méthoxy ou alcoyle de 1 à 3 atomes de carbone;

Y est H ou R';

R' est choisi parmi les groupes alcoyle de 1 à 4 atomes de carbone et alcanoyle de 1 à 12 atomes de carbone;

$R^2$ est choisi parmi l'hydrogène, le groupe hydroxy et les groupes alcanoyloxy de 1 à 4 atomes de carbone;

n est 1 ou 2; et

a est une double liaison en 6,7 quand n=1 et une liaison simple quand n=2;

et leurs sels pharmaceutiquement acceptables.

2. Composé selon la revendication 1 dans un véhicule pharmaceutique à raison de 0,5 à 95% en poids par rapport au poids total de la composition.

3. Composé selon la revendication 1, caractérisé en ce que R est un groupe cyclopropylméthyle.

4. Composé selon la revendication 1, caractérisé en ce que R est un groupe cyclobutylméthyle.

5. 17-méthyl-6,7-didéhydro-4,5-époxy-6-fluoro-3-méthoxymorphinane.

6. 17-méthyl-4,5-époxy-6,6-difluoro-3-méthoxymorphinane.

7. 17-méthyl-6,7-didéhydro-4,5-époxy-6-fluoro-3-méthoxymorphinane.

8. 17-méthyl-6,7-didéhydro-4,5-époxy-6-fluoro-3-hydroxymorphinane.

9. 17-cyclobutylméthyl-4,5-époxy-6,6-difluoro-3-méthoxymorphinane.

10. 17-cyclopropylméthyl-6,7-didéhydro-4,5-époxy-6-fluoro-3-acétoxymorphinane.

11. 17-cyclobutylméthyl-4,5α-époxy-6,6-difluoro-3-acétoxymorphinane.

12. 17-cyclopropylméthyl-4,5-époxy-6,6-difluoro-3-méthoxymorphinane.

13. 17-cyclopropylméthyl-6,7-didéhydro-4,5-époxy-6-fluoro-3-méthoxymorphinane.

14. 17-cyclopropylméthyl-4,5-époxy-6,6-difluoro-3-hydroxymorphinane.

15. 17-cyclopropylméthyl-6,7-didéhydro-4,5-époxy-6-fluoro-3-hydroxymorphinane.

16. 17-allyl-4,5-époxy-6,6-difluoro-3-méthoxymorphinane.

17. 17-allyl-6,7-didéhydro-4,5-époxy-6-fluoro-3-méthoxymorphinane.

18. Chlorhydrate de 17-cyclobutylméthyl-4,5α-époxy-6,6-difluoro-14β-hydroxy-3-méthoxymorphinane.

19. Chlorhydrate de 17-cyclopropylméthyl-4,5α-époxy-6,6-difluoro-14β-hydroxy-3-méthoxymorphinane.

20. Procédé selon lequel on fait réagir dans des conditions sensiblement anhydres la cétone de la formule

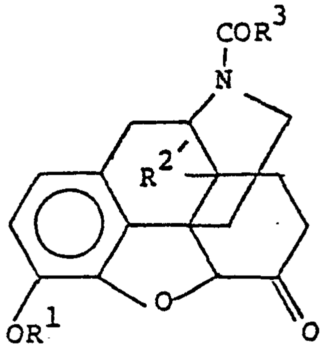

II

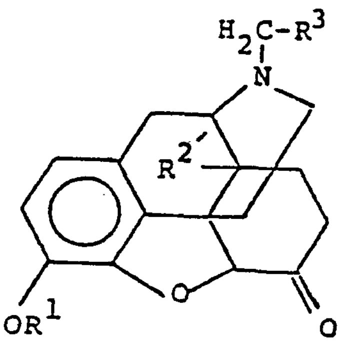

MA

III

dans laquelle

$R^1$ est un groupe alcoyle de 1 à 4 atomes de carbone ou alcanoyle de 1 à 12 atomes de carbone;

$R^2$ est l'hydrogène ou un groupe alcanoyloxy de 1 à 4 atomes de carbone;

$R^3$ est un groupe alcoyle de 1 à 9 atomes de carbone, vinyle, 1-propényle, isobuténylе, cycloalcoyle de 3 à 6 atomes de carbone, 2-furanyle, 2-tétrahydrofuranyle, 2-thiényle, 2-thiénylméthyle ou phénylméthyle qui peut être substitué au noyau par des substituents chloro, bromo, fluoro, méthoxy ou alcoyle de 1 à 3 atomes de carbone; et

MA est un acide minéral,

avec un agent de fluoration trifluorure d'aminosoufre disubstitué de la formule $R^4R^5NSF_3$ dans laquelle $R^4$ et $R^5$, identiques ou différents, sont chacun un groupe alcoyle primaire de 1 à 4 atomes de carbone; ou $R^4$ et $R^5$ quand ils sont pris ensemble sont $-(CH_2)_4-$, $-(CH_2)_5-$ ou $-CH_2CH_2OCH_2CH_2-$; à une température de −40°C à +80°C; en présence d'un solvant polaire ou non-polaire; et on recueille le composé fluoré de la formule

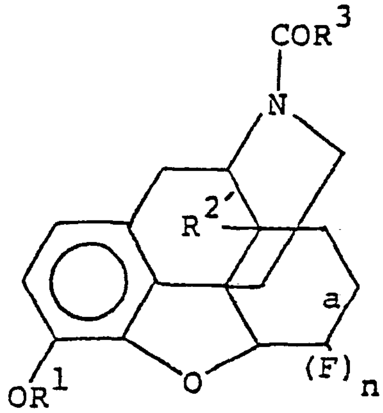

IV

quand la cétone est de formule II, et de la formule

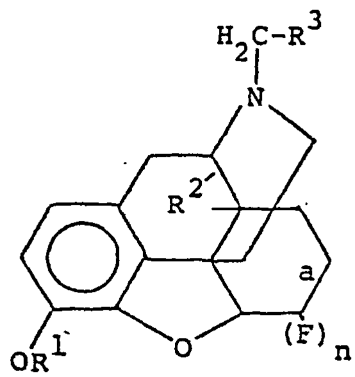

VII

quand la cétone est de formule III,
où dans les formules IV et VII
$R^1$, $R^{2'}$, $R^3$ et MA sont tels que définis ci-dessus;
n est 1 ou 2; et
a est une double liaison en 6,7 quand n=1 et une liaison simple quand n=2.

21. Procédé selon la revendication 20, caractérisé en ce qu'on fait réagir la cétone de formule III.

22. Procédé selon la revendication 20, caractérisé en ce qu'on fait réagir la cétone de formule II et que le composé fluoré ainsi produit est réduit pour transformation du groupe

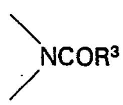

en groupe

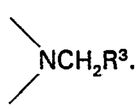

23. Procédé selon la revendication 22, caractérisé en ce que la réduction est effectuée avec $LiAlH_4$.

24. Procédé selon la revendication 20, caractérisé en ce que la cétone de départ est le chlorhydrate d'hydrocodone, l'agent de fluoration est le trifluorure de diéthylaminosoufre et le solvant est le solvant polaire éther méthylique de l'éthylène-glycol.

25. Procédé selon la revendication 20, caractérisé en ce que l'agent de fluoration est le trifluorure de diéthylaminosoufre et la solvant est le fluorotrichlorométhane.

26. Procédé selon la revendication 20, caractérisé en ce que l'agent de fluoration est le trifluorure de diéthylaminosoufre et le solvant est le solvant polaire chlorure de méthylène.

27. Procédé selon lequel on fait réagir dans des conditions sensiblement anhydres la cétone de la formule

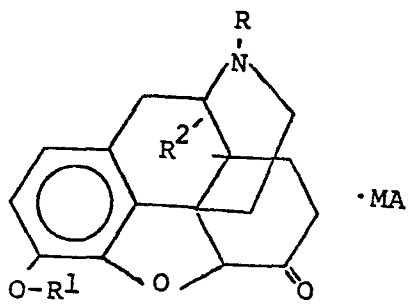

IA

dans laquelle

R est l'hydrogène, un groupe alcoyle de 1 à 10 atomes de carbone, allyle, méthylallyle, diméthylallyle, cycloalcoylméthyle de 4 à 7 atomes de carbone, 2-furanylméthyle, 2-tétrahydrofuranylméthyle, 2-thiénylméthyle, 2-thiényléthyle ou phényléthyle qui peut être substitué au noyau par des substituants chloro, bromo, fluoro, méthoxy ou alcoyle de 1 à 3 atomes de carbone;

$R^1$ est un groupe alcoyle de 1 à 4 atomes de carbone ou alcanoyle de 1 à 12 atomes de carbone;

$R^{2'}$ est l'hydrogène ou un groupe alcanoyloxy de 1 à 4 atomes de carbone; et

MA est un acide minéral,

avec un agent de fluoration trifluorure d'aminosoufre disubstitué de la formule $R^4R^5NSF_3$ dans laquelle $R^4$ et $R^5$, identiques ou différents, sont chacun un groupe alcoyle primaire de 1 à 4 atomes de carbone; ou $R^4$ et $R^5$ quand ils sont pris ensemble sont $-(CH_2)_4-$, $-(CH_2)_5-$ ou $-CH_2CH_2OCH_2CH_2-$; à une température de −40°C à +80°C; en présence d'un solvent polaire ou non-polaire; et on recueille le composé fluoré de la formule

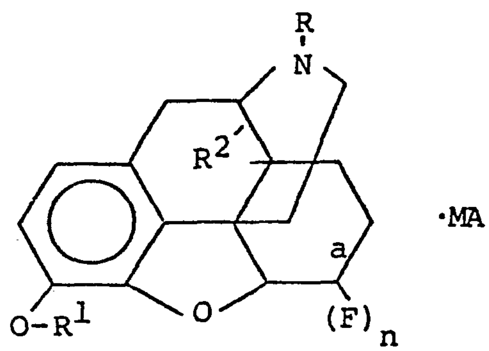

IB

dans laquelle

R, $R^1$ et $R^{2'}$ sont tels que définis ci-dessus;

n est 1 ou 2; et

a est une double liaison en 6,7 quand n=1 et une liaison simple quand n=2.

28. Composé de la formule

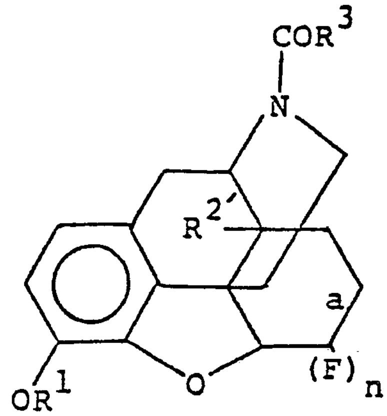

IV

dans laquelle

$R^1$ est un groupe alcoyle de 1 à 4 atomes de carbone ou alcanoyle de 1 à 12 atomes de carbone;

$R^{2'}$ est l'hydrogène ou un groupe alcanoyloxy de 1 à 4 atomes de carbone;

$R^3$ est un groupe alcoyle de 1 à 9 atomes de carbone, vinyle, 1-propényle, isobutényle, cycloalcoyle de 3 à 6 atomes de carbone, 2-furanyle, 2-tétrahydrofuranyle, 2-thiényle, 2-thiénylméthyle ou phénylméthyle qui peut être substitué au noyau par des substituants chloro, bromo, fluoro, méthoxy ou alcoyle de 1 à 3 atomes de carbone;

n est 1 ou 2; et

a est und double liaison en 6,7 quand n=1 et une liaison simple quand n=2.

**Patentansprüche**

1. Verbindung der Formel

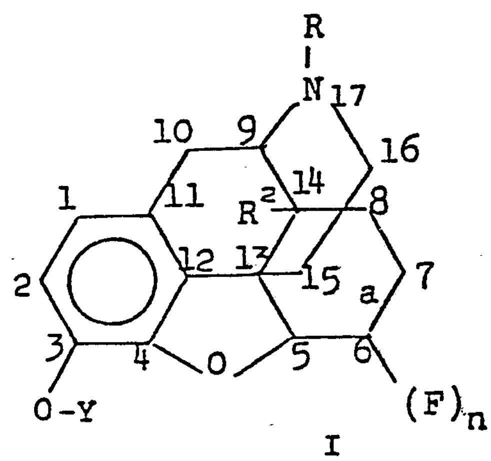

I

in der

R ausgewählt ist aus der aus Wasserstoff, Alkyl mit 1—10 Kohlenstoff-Atomen, Allyl, Methylallyl, Dimethylallyl, Cycloalkylmethyl mit 4—7 Kohlenstoff-Atomen, 2-Furanylmethyl, 2-Tetrahydrofuranylmethyl, 2-Thienylmethyl, 2-Thienylethyl und Phenylethyl, das am Ring durch Chloro, Bromo, Fluoro, Methoxy oder Alkyl-Substituenten mit 1—3 Kohlenstoff-Atomen substituiert sein kann, bestehenden Gruppe;

Y H oder R' ist;

R' ausgewählt ist aus der aus Alkyl mit 1—4 Kohlenstoff-Atomen und Alkanoyl mit 1—12 Kohlenstoff-Atomen bestehenden Gruppe;

$R^2$ ausgewählt ist aus der aus Wasserstoff, Hydroxy und Alkanoyloxy mit 1—4 Kohlenstoff-Atomen bestehenden Gruppe;

n 1 oder 2 ist und

a eine 6,7-Doppelbindung ist, wenn n=1, und eine Einfachbindung ist, wenn n=2,

und pharmazeutisch unbedenkliche Salze der Verbindung.

2. Verbindung nach Anspruch 1 in einem pharmazeutischen Träger in einer Menge von 0,5 bis 95

Gewichts-%, bezogen auf das Gesamtgewicht der Zusammensetzung.

3. Verbindung nach Anspruch 1, dadurch gekennzeichnet, daß R Cyclopropylmethyl ist.

4. Verbindung nach Anspruch 1, dadurch gekennzeichnet, daß R Cyclobutylmethyl ist.

5. 17-Methyl-6,7-didehydro-4,5-epoxy-6-fluoro-3-methoxymorphinan.

6. 17-Methyl-4,5-epoxy-6,6-difluoro-3-methoxymorphinan.

7. 17-Methyl-6,7-didehydro-4,5-epoxy-6-fluoro-3-methoxymorphinan.

8. 17-Methyl-6,7-didehydro-4,5-epoxy-6-fluoro-3-hydroxymorphinan.

9. 17-Cyclobutylmethyl-4,5-epoxy-6,6-difluoro-3-methoxymorphinan.

10. 17-Cyclopropylmethyl-6,7-didehydro-4,5-epoxy-6-fluoro-3-acetoxymorphinan.

11. 17-Cyclobutylmethyl-4,5α-epoxy-6,6-difluoro-3-acetoxymorphinan.

12. 17-Cyclopropylmethyl-4,5-epoxy-6,6-difluoro-3-methoxymorphinan.

13. 17-Cyclopropylmethyl-6,7-didehydro-4,5-epoxy-6-fluoro-3-methoxymorphinan.

14. 17-Cyclopropylmethyl-4,5-epoxy-6,6-difluoro-3-hydroxymorphinan.

15. 17-Cyclopropylmethyl-6,7-didehydro-4,5-epoxy-6-fluoro-3-hydroxymorphinan.

16. 17-Allyl-4,5-epoxy-6,6-difluoro-3-methoxymorphinan.

17. 17-Allyl-6,7-didehydro-4,5-epoxy-6-fluoro-3-methoxymorphinan.

18. 17 - Cyclobutylmethyl - 4,5α - epoxy - 6,6 - difluoro - 14β - hydroxy - 3 - methoxymorphinan hydrochlorid.

19. 17 - Cyclopropylmethyl - 4,5α - epoxy - 6,6 - difluoro - 14β - hydroxy - 3 - methoxymorphinan hydrochlorid.

20. Verfahren zur Umsetzung eines Ketons der Formel

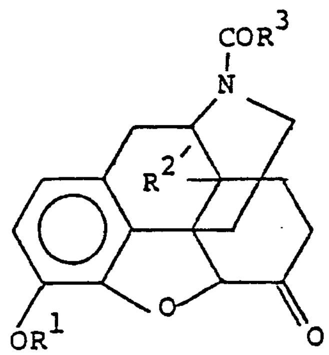

II

oder

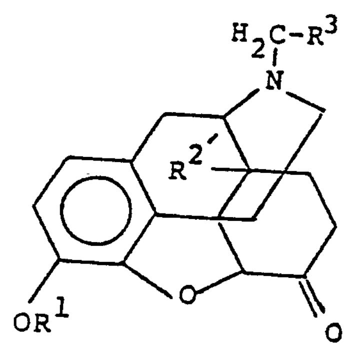

III

MA

in der

$R^1$ ein Alkyl mit 1—4 Kohlenstoff-Atomen oder ein Alkanoyl mit 1—12 Kohlenstoff-Atomen ist;

$R^{2'}$ Wasserstoff oder Alkanoyloxy mit 1—4 Kohlenstoffatom ist;

$R^3$ Alkyl mit 1—9 Kohlenstoff-Atomen, Vinyl, 1-Propenyl, Isobutenyl, Cycloalkyl mit 3—6 Kohlenstoff-Atomen, 2-Furanyl, 2-Tetrahydrofuranyl, 2-Thienyl, 2-Thienylmethyl oder Phenylmethyl, das am Ring durch Chloro, Bromo, Fluoro, Methoxy oder einen Alkyl-Substituenten mit 1—3 Kohlenstoff-Atomen substituiert sein kann, ist und

MA eine Mineralsäure ist,

unter im wesentlichen wasserfreien Bedingungen mit einem disubstituierten Aminoschwefeltrifluorid-Fluorierungsmittel der Formel $R^4R^5NSF_3$, in der jeweils $R^4$ und $R^5$, gleich oder voneinander verschieden, für eine primäre Alkyl-Gruppe mit 1—4 Kohlenstoff-Atomen stehen oder $R^4$ und $R^5$ zusammen genommen $-(CH_2)_4-$, $-(CH_2)_5-$ oder $-CH_2CH_2OCH_2CH_2-$ sind, bei einer Temperatur von −40°C bis +80°C in Gegenwart eines polaren oder unpolaren Lösungsmittels und Isolierung der fluorhaltigen Verbindung der Formel

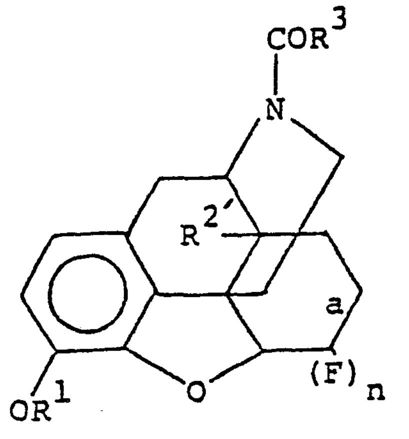

IV

wenn das Keton eine solches der Formel II ist, und der Formel

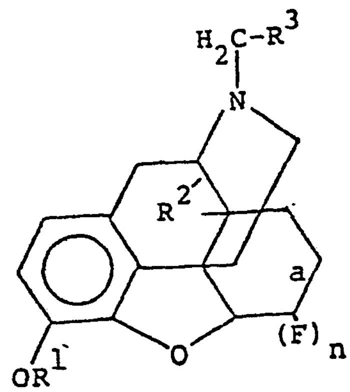

VII

wenn das Keton ein solches der Formel III ist, wobei in den angegebenen Formeln IV und VII $R^1$, $R^{2'}$, $R^3$ und MA die vorstehend angegebenen Bedeutungen haben, n 1 oder 2 ist und a eine 6,7-Doppelbindung ist, wenn n=1, und eine Einfachbindung ist, wenn n=2.

21. Verfahren nach Anspruch 20, dadurch gekennzeichnet, daß das Keton der Formel III umgesetzt wird.

22. Verfahren nach Anspruch 20, dadurch gekennzeichnet, daß das Keton der Formel II umgesetzt und die dadurch hergestellte fluorhaltige Verbindung reduziert wird, um die Gruppe

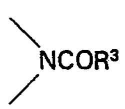

in die Gruppe

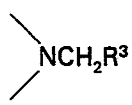

zu überführen.

23. Verfahren nach Anspruch 22, dadurch gekennzeichnet, daß die Reduktion mit $LiAlH_4$ durchgeführt wird.

24. Verfahren nach Anspruch 20, dadurchgekennzeichnet, daß das Ausgangsketon Hydrocodonhydrochlorid ist, das Fluorierungsmittel Diethylaminoschwefeltrifluorid ist und das Lösungsmittel das polare Lösungsmittel Ethylenglycol-methylether ist.

25. Verfahren nach Anspruch 20, dadurch gekennzeichnet, daß das Fluorierungsmittel Diethylaminoschwefeltrifluorid ist und das Lösungsmittel Fluorotrichloromethan ist.

26. Verfahren nach Anspruch 20, dadurch gekennzeichnet, daß das Fluorierungsmittel Diethylaminoschwefeltrifluorid ist und das Lösungsmittel das polare Lösungsmittel Methylenchlorid ist.

27. Verfahren zur Umsetzung eines Ketons der Formel

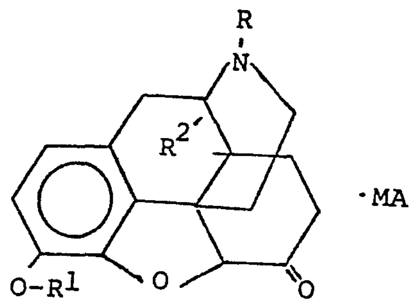

IA

in der

R Wasserstoff, Alkyl mit 1—10 Kohlenstoff-Atomen, Allyl, Methylallyl, Dimethylallyl, Cycloalkylmethyl mit 4—7 Kohlenstoff-Atomen, 2-Furanylmethyl, 2-Tetrahydrofuranylmethyl, 2-Thienylmethyl, 2-Thienylethyl oder Phenylethyl, das am Ring durch Chloro, Bromo, Fluoro, Methoxy oder einen Alkyl-Substituenten mit 1—3 Kohlenstoff-Atomen substituiert sein kann, ist;

$R^1$ ein Alkyl mit 1—4 Kohlenstoff-Atomen oder eine Alkanoyl mit 1—12 Kohlenstoff-Atomen ist;

$R^2$ Wasserstoff oder Alkanoyloxy mit 1—4 Kohlenstoffatomen ist und

MA eine Mineralsäure ist,

unter im wesentlichen wasserfreien Bedingungen mit einem disubstituierten Aminoschwefeltrifluorid-Fluorierungsmittel der Formel $R^4R^5NSF_3$, in der jeweils $R^4$ und $R^5$, gleich oder voneinander verschieden, für eine primäre Alkyl-Gruppe mit 1—4 Kohlenstoff-Atomen stehen oder $R^4$ und $R^5$ zusammen genommen $—(CH_2)_4—$, $—(CH_2)_5—$ oder $—CH_2CH_2OCH_2CH_2—$ sind, bei einer Temperatur von −40°C bis +80°C in Gegenwart eines polaren oder unpolaren Lösungsmittels und Isolierung der fluorhaltigen Verbindung der Formel

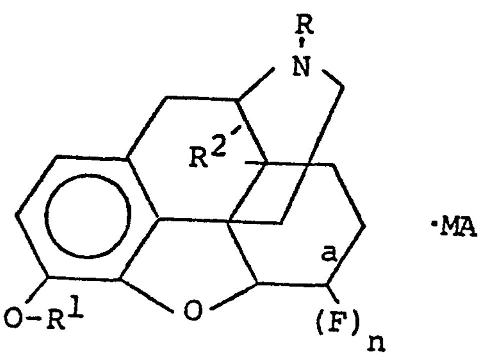

IB

in der

R, $R^1$ und $R^{2'}$ die vorstehend angegebenen Bedeutungen haben, n 1 oder 2 ist und a eine 6,7-Doppelbindung ist, wenn n=1, und ein eine Einfachbindung ist, wenn n=2.

28. Verbindung der Formel

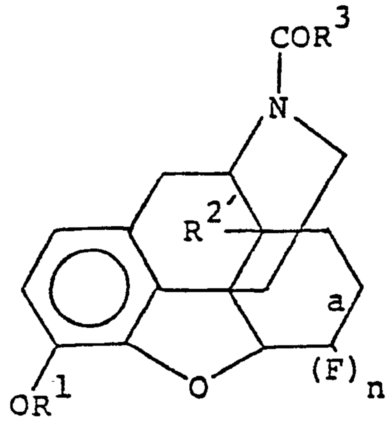

IV

in der

$R^1$ Alkyl mit 1—4 Kohlenstoff-Atomen oder Alkanoyl mit 1—12 Kohlenstoff-Atomen ist;

$R^{2'}$ Wasserstoff oder Alkanoyloxy mit 1—4 Kohlenstoff-Atomen ist;

$R^3$ Alkyl mit 1—9 Kohlenstoff-Atomen, Vinyl, 1-Propenyl, Isobutenyl, Cycloalkyl mit 3—6 Kohlenstoff-Atomen, 2-Furanyl, 2-Tetrahydrofuranyl, 2-Thienyl, 2-Thienylmethyl oder Phenylmethyl, das am Ring durch Chloro, Bromo, Fluoro, Methoxy oder einen Alkyl-Substituenten mit 1—3 Kohlenstoff-Atomen substituiert sein kann, ist;

n 1 oder 2 ist und

a eine 6,7-Doppelbindung ist, wenn n=1, und eine Einfachbindung ist, wenn n=2.